# EUROPEAN PATENT APPLICATION

(11) **EP 4 202 062 A1**
(43) Date of publication of application: **28.06.2023**
(21) Application number: 21857767.4
(22) Date of filing: 20.08.2021
(51) Int. Cl.: C12Q 1/6886, A61K 38/00, C12N 15/63, A61P 37/00, A61P 35/00, A61P 25/00, A61P 7/00

(54) **USE OF MCM8-CGAS-STING-I-TYPE INTERFERON SIGNAL PATHWAY AS DISEASE TARGET**

(30) Priority: 21.08.2020 CN 202010847559
(71) Applicant: Guangzhou Women And Children's Medical Center, Guangzhou, Guangdong 510000 (CN)
(72) Inventor: ZHANG, Yuxia, Guangzhou, Guangdong 510000 (CN); LIN, Meng, Guangzhou, Guangdong 510000 (CN); XIAN, Huifang, Guangzhou, Guangdong 510000 (CN); CHEN, Zhanghua, Guangzhou, Guangdong 510000 (CN); BAI, Fan, Guangzhou, Guangdong 510000 (CN); CUI, Jun, Guangzhou, Guangdong 510000 (CN); GU, Xiaoqiong, Guangzhou, Guangdong 510000 (CN)
(74) Representative: Gassner, Birgitta
(86) International application number: PCT/CN2021/113811
(87) International publication number: WO 2022/037679

(57) **Abstract**

The present disclosure relates to the field of biomedicine, in particular to the use of MCM8-cGAS-STING-IFN-I signal pathway as a disease target, including the use in the preparation of an animal model of a disease or the preparation of a product for the diagnosis, prevention, or treatment of a disease. In particular, the present disclosure comprises the use of a reagent for quantitatively detecting the gene expression or protein expression or protein activity of MCM8 in the preparation of a product for the diagnosis of a disease, wherein the disease is a disease caused by dysfunctional mitophagy or a disease caused by abnormal activation of cGAS-STING-IFN-I signaling pathway; if the gene expression or protein expression or protein activity of MCM8 is lower than a reference level, the disease is diagnosed; and the reference level represents the gene expression level or protein expression level or protein activity level of MCM8 in a subject in the same range of age who does not have the disease.

## Description

### TECHNICAL FIELD

The present disclosure relates to the biomedical field, in particular to the use of MCM8-cGAS-STING-IFN-I signal pathway as disease target and more particularly to the use of MCM8 and the related signaling pathway or targets in the pathway (including TRIM21, mitochondrial LC3, and cGAS-STING-IFN-I) as a target for the diagnosis and treatment of vasculitis diseases such as Kawasaki disease and other diseases caused by the abnormalities in the signal pathway.

### BACKGROUND

Kawasaki disease, also known as mucocutaneous lymph node syndrome (MCLS), is an acute febrile eruptive disease in children with systemic vasculitis lesions as the main pathology. Kawasaki disease is mostly found in infants and young children, with a high incidence in East Asia, possibly accompanied by severe complications. Up to 25% of Kawasaki disease instances may cause coronary artery injury, and in severe cases, it may cause thrombotic infarction, myocardial infarction and coronary artery aneurysm. In developed countries, Kawasaki disease has become the main cause for inducing acquired heart disease. The main diagnostic criteria for Kawasaki disease involve persistent fever accompanied by skin rash, swollen cervical lymph nodes, conjunctival congestion, oral symptoms (such as strawberry tongue, cracked lips and mucosal congestion), and changes in extremities (such as palm swelling, erythema palmatum, and excoriation). According to the guidance of the American Heart Association, Kawasaki disease can be diagnosed if the fever lasts for more than 5 days or for 4 days with at least 4 of the above complications. At present, the etiology of Kawasaki disease remains unclear, but the mainstream view believes that Kawasaki disease is determined by both infectious and genetic factors (Onouchi Y, Circulation Journal 2012, 76(7): 1581-1586).

Kawasaki disease occurs more frequently in winter and spring and has regional outbreaks, presenting epidemiological characteristics, suggesting that infection with pathogens may be the main inducement of Kawasaki disease. However, the specific cause of Kawasaki disease is yet unclear to date. The researches of two experimental groups of Turnier JL et al. and Chang LY et al. show that half of children with Kawasaki disease had been infected with at least one respiratory virus (Turnier JL et al., Pediatrics 2015, 136(3): E609-E614; Chang LY et al., J Formos MedAssoc 2014, 113(3): 148-154), suggesting that viruses may be one of the important inducements of Kawasaki disease. Rowley AH et al. also detect respiratory virus RNA in the ultra-microstructure study on autopsy specimens of Kawasaki disease (Rowley AH et al., Nat Rev Microbiol 2008, 6(5): 394-401.). Viruses including coxsackievirus, parainfluenza virus, respiratory syncytial virus, metapneumovirus, Chikungunya fever virus, cytomegalovirus, etc. may be the inducement of Kawasaki disease. In addition, Corona Virus Disease 2019 (COVID-19) may also induce systemic lesions similar to Kawasaki disease. In addition to viruses, bacteria may also be a potential cause of Kawasaki disease. There are viewpoints that several superantigens from bacteria, including streptococcal pyrogenic exotoxin SPE-A, toxic shock syndrome toxin TSST-1 and staphylococcal enterotoxin, can aggregate Vb regions of T cell receptors and stimulate T cells to produce a large amount of inflammatory factors.

The incidence of Kawasaki disease in East Asia populations is 10-20 times higher than that in other regions, and the incidence in East Asians who emigrated to European and American countries is still significantly higher than that in other populations, suggesting the genetic susceptibility. Burgner D et al. early found that FCGR2A was related to the susceptibility to Kawasaki disease through GWAS (Burgner D et al., PLoS Genet. 2009 Jan; 5(1): e1000319). Recently, Onouchi Y et al. found that ITPKC gene mutation can regulate the release of proinflammatory factors such as IL-2 and excessive activation of T cells, thus increasing the susceptibility to Kawasaki disease (Onouchi Y et al., Nature genetics 2008, 40(1): 35-42.). Later, Alphonse MP et al. reports again that ITPKC affects the pathological development of Kawasaki disease by involvement in the regulation of the activation of NLRP3 inflammasome and the release of inflammatory factors IL-1b and IL-18 (Alphonse MP et al., Journal of immunology 2016, 197(9): 3481-3489.). However, more susceptibility genes, especially those specific to East Asian populations, are yet to be discovered.

There is an important unmet need for a solution for treatment of Kawasaki disease as a systemic vasculitis. Similar to other vasculitis, typical treatments for Kawasaki disease involve administration of glucocorticoids and aspirin, an anti-platelet aggregation drug, which usually takes a long period for administration. The treatment depends on the nature and degree of the progression of the disease and may include non-steroidal anti-inflammatory agents, antihistamines, cytotoxic drugs such as cyclophosphamide, or immunosuppressants such as methotrexate. Many of these agents have obvious side effects, especially after taking for a long time, such as bone marrow suppression (leukopenia and thrombocytopenia), secondary malignant disease, infertility, pulmonary interstitial fibrosis, infection, and steroid-induced diabet, which are worse than the primary disease itself in some cases. At present, the standard treatment method for Kawasaki disease is injection of immunoglobulin IVIG combined with aspirin. Timely treatment with globulin and aspirin can significantly reduce the incidence of coronary artery complications. However, 10-20% of children with Kawasaki disease do not respond to traditional treatment methods, and thus it is necessary to actively explore new and less drug-resistant treatment methods or effective multi-target combination treatment methods.

In addition, Kawasaki disease, as a systemic vasculitis, has inflammatory lesions in many blood vessels of the whole body, especially in cardiovascular system, and is a representative refractory vasculitis. Therefore, generally, drugs that can treat Kawasaki disease may also have certain effects on treating other vasculitis. Therefore, the present disclosure concerns Kawasaki disease as well as other vasculitis.

Vasculitis, also known as angiitis, is a class of diseases with inflammatory cell infiltration on vascular walls and around blood vessels, accompanied by vascular injury, including cellulose deposition, collagen fibrosis, and endothelial cell and muscle cell necrosis. The cause of vasculitis is unclear. It is generally believed that serum disease, drug allergy and virus infection can cause vasculitis.

Vasculitis is subdivided into three categories associated with the size of the affected blood vessels (small blood vessels, medium blood vessels and large blood vessels), among which vasculitis of large blood vessels includes aorto-arteritis and giant cell arteritis. Vasculitis of medium blood vessels includes polyarteritis nodosa and Kawasaki disease. Vasculitis of small blood vessels includes Wegener's granulomatosis, microscopic polyangiitis, anaphylactoid purpura and cutaneous leukocytoclastic vasculitis. Behcet's disease may be involved in all of the large, medium and small arteries and veins. In addition, microangiitis, or vasculitis of both arteries and veins, is also included, including, for example, intraglomerular microangiitis, which is the characteristic of various kidney diseases, or thromboangiitis obliterans and superficial phlebitis.

Other classifications for vasculitis mainly include: (1) allergic leukocytoclastic (necrotizing) vasculitis caused by allergy (mainly including allergic cutaneous vasculitis, allergic systemic vasculitis, anaphylactoid purpura, hypocomplementemic (urticaria-like) vasculitis, etc.); (2) polyarteritis nodosa (mainly including systemic polyarteritis nodosa, benign cutaneous polyarteritis nodosa, infantile polyarteritis nodosa, etc.); (3) thrombotic vasculitis (mainly including thromboangiitis obliterans, thrombophlebitis, malignant atrophic papulosis, livedoreticularis vasculitis, thrombotic thrombocytopenic purpura, etc.); (4) granulomatous vasculitis (mainly including Wegener's granulomatosis, allergic granulomatous vasculitis, temporal arteritis, Takayasu's arteritis, etc.); (5) lymphocytic vasculitis (mainly including lymphomatoid papulosis, pityriasis lichenoides et varioliformis acute, etc.); (6) nodular including vasculitis (mainly nodular vasculitis and erythema induratum); and (7) vasculitis with abnormal blood composition (mainly including cryoglobulinemia, hypercryoglobulinemia, macroglobulinemia, etc.).

Other classifications of vasculitis further include those named depending on the location of vasculitis, including but not limited to cutaneous vasculitis, visceral vasculitis (heart, liver, spleen, lung, kidney, etc.), cerebral vasculitis, etc. As classified based on the role of pathogenic factors, vasculitis caused by pathogenic factors directly acting on blood vessel walls is primary vasculitis, while vasculitis caused by inflammatory lesions in adjacent tissues spreading to blood vessel walls is secondary vasculitis.

In addition, systemic lupus erythematosus, also known as lupus vasculitis, has a pathological basis similar to Kawasaki disease and is also a kind of systemic vasculitis where the permeability of blood vessels increases and inflammatory manifestations in visceral organs occur or Raynaud's phenomenon appears. It is an autoimmune connective tissue disease involving multiple organs such as skin, joints, serosa, kidney, heart and blood vessels, and nervous centralis.

Minichromosome maintenance deficient 8 gene, or MCM8 for short, is a member of the DNA helicase family. The nuclear activities of MCM8 include nucleotide binding, DNA binding, ATP binding, DNA-dependent ATPase, and nucleoside triphosphatase. Cellular functions involved by MCM8 include DNA replication, DNA replication initiation/transcription/regulation of transcription, DNA dependence/cell cycle, etc. The diseases caused by the defects in the gene include gastric cancer, infertility associated with ovarian hypoplasia, etc.; however, the mechanism of how this gene is associated with the diseases is unknown, and at present, there is no report about the relationship between MCM8 gene and vasculitis (including Kawasaki disease, systemic lupus erythematosus, etc.), cataract, fatty liver, pneumonia or severe pneumonia, nephritis and other diseases either. It would be contributing to provide much more, more complete and more accurate methods for the targeted diagnosis, prevention and/or treatment of related diseases by clarifying the role of MCM8 gene in signaling pathway and the connection of MCM8 gene with the occurrence and development of diseases.

### SUMMARY

The present disclosure aims to solve at least some of the above technical problems, and to diagnose, prevent and/or treat a disease caused by abnormal activation of cGAS-STING-IFN-I signaling pathway or a disease caused by dysfunctional mitophagy by the detection/regulation of the expression of MCM8 and matters in its related pathways.

In one aspect of the present disclosure, provided is the use of a reagent for quantitatively detecting a gene expression or protein expression or protein activity of MCM8 in the preparation of a product for the diagnosis of a disease, wherein the disease is a disease caused by dysfunctional mitophagy and/or a disease caused by abnormal activation of cGAS-STING-IFN-I signaling pathway; wherein if the gene expression or protein expression or protein activity of MCM8 is lower than a reference level, the disease is diagnosed; and the reference level represents the gene expression level or protein expression level or protein activity level of MCM8 in a subject in the same range of age who does not have the disease.

Preferably, the product comprises a kit, a diagnostic reagent, a detection reagent, a gene chip or a protein chip.

Preferably, the protein activity is preferably a binding activity of MCM8 protein to TRIM21 protein or a binding activity of MCM8 protein to mitochondrial LC3 protein. More preferably, when detecting the binding activity of MCM8 protein to TRIM21 protein, the detection method for the binding activity is to detect the ubiquitination level of MCM8 protein; when detecting the binding activity of MCM8 protein to mitochondrial LC3 protein, the detection method for the binding activity is to detect the binding activity of LIR motif sequence with the amino acid sequence RSFTAL (SEQ ID NO: 4) in MCM8 protein to mitochondrial LC3 protein.

Preferably, the product is configured for performing one or more of the detection methods selected from the group consisting of: polymerase chain reaction, micro digital polymerase chain reaction, fluorescence polymerase chain reaction, loop-mediated isothermal amplification reaction, enzyme-linked immunosorbent analysis, nucleotide or amino acid sequence sequencing, denaturing gradient gel electrophoresis, nucleic acid typing chip detection, high performance liquid chromatography, in-situ hybridization, biological mass spectrometry, high-resolution melting curve analysis, single-strand conformational isomerism polymorphism analysis, or probe amplification-refractory mutation system analysis.

Preferably, the product contains a primer and/or detection probe for detecting MCM8 gene and/or control gene; and/or further contains a sample treatment reagent, including but not limited to a sample lysis reagent, a sample purification reagent and/or a sample nucleic acid extraction reagent; and/or further contains one or more of DNA extraction reagent, dNTP, DNA polymerase, double-strand specific fluorescent dye, and water.

Preferably, the product is used for a detection specimen or test sample comprising one or more of blood, body fluid, urine, tissue, and cell samples from a subject to be tested; the subject to be tested is an adult or a child, preferably a child.

In a second aspect of the present disclosure, provided is the use of MCM8 gene-knockout mice in the preparation of an animal model of disease, wherein the disease is a disease caused by dysfunctional mitophagy and/or a disease caused by abnormal activation of cGAS-STING-IFN-I signaling pathway.

Preferably, the preparation method for gene knockout mice involves using CRISPR/cas9 technology to construct MCM8 knockout mice, including MCM8+/- heterozygous mice or MCM8-/- homozygous mice, preferably MCM8-/- homozygous mice.

Preferably, 32-week-old gene-knockout mice are selected as a spontaneous animal model of the disease.

In a third aspect of the present disclosure, provided is the use of a substance for improving a gene expression or protein expression or protein activity of MCM8 in the preparation of a drug for the prevention and/or treatment of a disease, wherein the disease is a disease caused by dysfunctional mitophagy and/or a disease caused by abnormal activation of cGAS-STING-IFN-I signaling pathway.

Preferably, the protein activity is a binding activity of MCM8 protein to TRIM21 protein or a binding activity of MCM8 protein to mitochondrial LC3 protein.

Preferably, the substance for improving a gene expression or protein expression or protein activity of MCM8 is selected from the group consisting of a micromolecular drug, a polysaccharide drug, a nucleic acid drug, and a polypeptide or protein drug. More preferably, the substance is one or more of a nucleic acid drug containing an MCM8 expression promoter or enhancer, a nucleic acid drug containing the full-length mRNA sequence of MCM8 (e.g., the sequence as shown in SEQ ID NO: 1 and other mRNA isoform sequences of the gene) or a DNA sequence in which U in the full-length mRNA is replaced by T, or a complementary sequence thereto, a nucleic acid drug having any truncated sequence (e.g., SEQ ID NO: 5 and SEQ ID NO: 6) of MCM8 mRNA containing at least a nucleotide sequence shown in SEQ ID NO: 3 or a DNA sequence in which U in the mRNA is replaced by T, or a complementary sequence thereto, a polypeptide or protein drug having any truncated (e.g., SEQ ID NO: 7 and SEQ ID NO: 8) or full-length sequence of MCM8 protein (e.g., the sequence as shown in SEQ ID NO: 2 and other amino acid isoform sequences of the protein) containing at least an amino acid sequence shown in SEQ ID NO: 4, and a molecular glue capable of binding MCM8 protein to mitochondrial LC3 protein.

Preferably, the drug is prepared in a doe form suitable for adults or children, and/or a dose form suitable for gastrointestinal, transdermal, ocular, nasal, or pulmonary administration.

The dose form suitable for adults are various pharmaceutical dose forms prepared by using the pharmaceutical active ingredients of the present disclosure and conventional excipients in the art through conventional methods in the art, and the pharmaceutical dose forms comprise but are not limited to powders, tablets, sublingual tablets, granules, capsules, solutions, emulsions, suspensions, injections, suppositories, aerosols, effervescent tablets, drops, dripping pills, eye drops, eye ointments, skin patches, etc.

More preferably, the drug is prepared in a dose form suitable for children, and the dose form suitable for children comprise granules, syrups, chewable tablets, dispersible tablets, suspensions, effervescent tablets, drops, enteric-coated agents, enemas, orally disintegrating tablets, solutions, electuaries, and suppositories.

The drug may also be prepared into liposomes or nano-liposomes, fine powders, or freeze-dried powders, microemulsions, eye drops or eye ointments, solutions such as oral solutions and injections, liposomes, transdermal absorption preparations such as ointments or patches or gels, nasal drops, pulmonary inhalation preparations such as inhalation powders and sustained and controlled release preparations such as microsphere injections and implants depending on the requirements for pharmaceutical ingredients.

In a fourth aspect of the present disclosure, provided is a pharmaceutical composition for the prevention and/or treatment of a disease, wherein the disease is a disease caused by dysfunctional mitophagy or a disease caused by abnormal activation of cGAS-STING-IFN-I signaling pathway, and the pharmaceutical composition contains a combination of a) and c) or a combination of a), b) and c):
a) one of or any combination of a drug for improving the gene expression of MCM8, a drug for improving the protein expression of MCM8, a drug for improving the binding of MCM8 protein to TRIM21 protein, a drug for improving the ubiquitination or phosphorylation modification of MCM8 in nucleus, and a drug for improving the binding of MCM8 protein to mitochondrial LC3 protein,
   wherein a) is selected from the group consisting of a micromolecular drug, a polysaccharide drug, a nucleic acid drug, and a polypeptide or protein drug; more preferably, a) is a nucleic acid drug containing an MCM8 expression promoter or enhancer, a nucleic acid drug containing the full-length mRNA sequence of MCM8 (e.g., the sequence as shown in SEQ ID NO: 1 and other mRNA isoform sequences of the gene) or a DNA sequence in which U in the full-length mRNA is replaced by T, or a complementary sequence thereto, a nucleic acid drug having any truncated sequence (e.g., SEQ ID NO: 5 and SEQ ID NO: 6) of MCM8 mRNA containing at least a nucleotide sequence shown in SEQ ID NO: 3 or a DNA sequence in which U in the mRNA is replaced by T, or a complementary sequence thereto, a polypeptide or protein drug having any truncated (e.g., SEQ ID NO: 7 and SEQ ID NO: 8) or full-length sequence of MCM8 protein (e.g., a sequence as shown in SEQ ID NO: 2 and other amino acid isoform sequences of the protein) containing at least an amino acid sequence shown in SEQ ID NO: 4, and a molecular glue capable of binding MCM8 protein to mitochondrial LC3 protein;
b) one of or any combination of a drug for reducing mitochondrial DNA aggregation in cytoplasm, a drug for inhibiting cGAS-STING signaling pathway, a drug for inhibiting Interferon-I (IFN-I) expression or binding to a receptor thereof, and other active drugs capable of preventing or treating the disease,
   wherein b), when selected from a drug for inhibiting IFN-I expression or binding to a receptor thereof, is a neutralizing antibody against IFN-I including Interferon α (IFN-α) and/or Interferon β (IFN-β), a blocking antibody against IFN-I receptor, or a drug stimulating a subject to produce an antibody against IFN-I; more preferably, the antibody is one or more of Anifrolumab, Faralimomab, MEDI-545, Rontalizumab, Sifalimumab, AGS-009, IFNα Kinoid, NI-0101, and 1-401; and
c) a delivery vector for a nucleic acid, polypeptide, or protein, and/or a pharmaceutically acceptable carrier.

In a fifth aspect of the present disclosure, provided is a diagnostic marker for a disease, wherein the disease is a disease caused by dysfunctional mitophagy or a disease caused by abnormal activation of cGAS-STING-IFN-I signaling pathway; the diagnostic marker is a mutant MCM8 gene or MCM8 protein; the mutant MCM8 gene has a decreased expression level relative to a reference level, or the mutant MCM8 protein has a decreased expression level relative to a reference level, or the mutant MCM8 protein has a decreased binding activity to TRIM21 protein relative to a reference level, or the mutant MCM8 protein has a decreased binding activity to mitochondrial LC3 protein relative to a reference level; the reference level represents the gene expression level or protein expression level or protein activity level of MCM8 in a subject in the same range of age who does not have the disease.

Preferably, the disease caused by dysfunctional mitophagy and/or the disease caused by abnormal activation of cGAS-STING-IFN-I signaling pathway are selected from the group consisting of autoimmune diseases, inflammatory diseases, neurological conditions, aging-related diseases or cancers.

The disease caused by dysfunctional mitophagy is preferably cardiovascular disease, neurological disease or neurodegenerative disease, or cancer; and the disease caused by abnormal activation of cGAS-STING-IFN-I signaling pathway comprises autoimmune diseases, inflammatory diseases, cancers etc., such as Aicardi-Goutieres syndrome, and systemic lupus erythematosus, preferably vasculitis, severe pneumonia, nephritis, systemic lupus erythematosus, fatty liver (including alcoholic fatty liver or nonalcoholic fatty liver) and cataract.

In a sixth aspect of the present disclosure, provided is a diagnostic marker for vasculitis, wherein the diagnostic marker is MCM8 gene with 826G>C and/or 1094G>A and/or 839C>A and/or 2291C>G mutations, or MCM8 protein with A276P and/or S365N and/or S280C and/or S764A mutations, or other MCM8 protein single mutations or linked mutations except A276P mutation present at positions 272-277 of the MCM8 protein sequence of SEQ ID NO: 2, or gene mutations or linked mutations at the corresponding sites (other MCM8 gene single mutations or linked mutations except 826G>C mutation present at positions 816-831 of the MCM8 gene sequence of SEQ ID NO: 1).

Preferably, the vasculitis is Kawasaki disease or systemic lupus erythematosus.

In a seventh aspect of the present disclosure, provided is the use of the diagnostic marker for vasculitis in the preparation of a product for detecting vasculitis, wherein the mutant is MCM8 gene with 826G>C and/or 1094G>A and/or 839C>A and/or 2291C>G mutations, or MCM8 protein with A276P and/or S365N and/or S280C and/or S764A mutations; or other MCM8 protein single mutations or linked mutations except A276P mutation present at positions 272-277 of the MCM8 protein sequence of SEQ ID NO: 2, or gene mutations or linked mutations at the corresponding sites (other MCM8 gene single mutations or linked mutations except 826G>C mutation present at positions 816-831 of the MCM8 gene sequence of SEQ ID NO: 1).

Preferably, the vasculitis is Kawasaki disease or systemic lupus erythematosus.

Preferably, the product comprises a kit, a diagnostic reagent, a detection reagent, a gene chip or a protein chip.

Preferably, the product is configured for performing one or more of the detection methods selected from the group consisting of: polymerase chain reaction, micro digital polymerase chain reaction, fluorescence polymerase chain reaction, loop-mediated isothermal amplification reaction, enzyme-linked immunosorbent assay (ELISA), nucleotide or amino acid sequence sequencing, denaturing gradient gel electrophoresis, nucleic acid typing chip detection, high performance liquid chromatography, in-situ hybridization, biological mass spectrometry, high-resolution melting curve analysis, single-strand conformational isomerism polymorphism analysis, or probe amplification-refractory mutation system analysis.

Preferably, the product contains a primer for detecting MCM8 gene and/or control gene; and/or a probe for detecting MCM8 gene mutation or control gene; and/or further contains a sample treatment reagent, including but not limited to a sample lysis reagent, a sample purification reagent and/or a sample nucleic acid extraction reagent; and/or further contains one or more of DNA extraction reagent, dNTP, DNA polymerase, double-strand specific fluorescent dye, and water.

Preferably, the product is used for a detection specimen or test sample comprising one or more of blood, body fluid, urine, tissue, and cell samples from a subject to be tested; the subject to be tested is an adult or a child, preferably a child.

In an eighth aspect of the present disclosure, provided is a method for the diagnosis or detection of vasculitis, the method comprising detecting whether there is a mutation site in the MCM8 gene or the encoded protein in a subject. If there is a mutation site, whether homozygous or heterozygous, as long as the mutation site affects the function of the gene or the protein, for example, making the expression or activity level of MCM8 protein decrease relative to the level in populations at the same age who do not have vasculitis, then the subject is identified as having vasculitis, or the subject is diagnosed as being at a high risk for vasculitis, or else the subject is diagnosed such that whose offspring easily carries vasculitis susceptibility genes. Preferably, the vasculitis is Kawasaki disease or systemic lupus erythematosus; and wherein the subject may be any population such as children, adults, and the elderly. The mutation site may be any mutation on the gene or protein that satisfies the above definitions, for example, a mutation in the LIR motif sequence AGGUCAUUUACUGCUCUC (SEQ ID NO: 3, and a DNA sequence in which U in the mRNA is replaced by T, or a complementary sequence thereto) of the MCM8 gene sequence or a mutation in the LIR motif sequence RSFTAL (SEQ ID NO: 4) of the MCM8 protein sequence; for another example, the mutation discovered by the inventors as described in the present disclosure hereinbefore can be selected, and the mutation is MCM8 gene with 826G>C and/or 1094G>A and/or 839C>A and/or 2291C>G mutations (that is, in SEQ ID NO: 1, guanine G at position 826 is mutated into cytosine C; and/or guanine G at position 1094 is mutated into adenine A; and/or cytosine C at position 839 is mutated into adenine A; and/or cytosine C at position 2291 is mutated into guanine G), or the mutation is MCM8 protein with A276P and/or S365N and/or S280C and/or S764A mutations (that is, in SEQ ID NO: 2, alanine A at position 276 is mutated into proline P; and/or serine S at position 365 is mutated into asparagine N; and/or serine S at position 280 is mutated into cysteine C; and/or serine S at position 764 is mutated into alanine A).

Preferably, the method for the diagnosis or detection of vasculitis of the present disclosure comprises a step of detection with a primer and/or probe, and the fragments amplified by the primer and/or probe contain the whole gene or include at least fragments by which whether there is a target mutation site can be detected. In addition, in the method for detection of vasculitis of the present disclosure, the mutation site is detected by a method or technique selected from the group consisting of: polymerase chain reaction, micro digital polymerase chain reaction, fluorescence polymerase chain reaction, loop-mediated isothermal amplification reaction, enzyme-linked immunosorbent assay, nucleotide or amino acid sequence sequencing, denaturing gradient gel electrophoresis, nucleic acid typing chip detection, high performance liquid chromatography, in-situ hybridization, biological mass spectrometry, high-resolution melting curve analysis, single-strand conformational isomerism polymorphism analysis, and probe amplification-refractory mutation system.

In a ninth aspect of the present disclosure, provided is the use of MCM8 gene-knockout mice in the preparation of an animal model of vasculitis.

Preferably, the vasculitis is Kawasaki disease or systemic lupus erythematosus.

Preferably, when the vasculitis is Kawasaki disease, the use comprises the step of: constructing Kawasaki disease mice model by intraperitoneal injection of LCWE (*Lactobacillus casei* cell wall extract) into MCM8 knockout mice constructed by a conventional method in the art; and when the vasculitis is systemic lupus erythematosus, the use comprises the steps of: constructing lupus nephritis mice model of by intraperitoneal injection of pristane into MCM8 knockout mice constructed by conventional method in the art.

In a tenth aspect of the present disclosure, provided is the use of MCM8 gene and/or protein as a drug target for the preparation of a drug for the prevention and/or treatment of vasculitis. The use comprises, without limitation, preparing any substance that capable of improving the normal gene expression or protein expression or protein activity of MCM8 as an active ingredient, and a nucleic acid, polypeptide or protein delivery vector that capable of delivering the active ingredient, and/or a pharmaceutically acceptable carrier into a conventional dosage form in the art.

Preferably, the vasculitis is Kawasaki disease or systemic lupus erythematosus.

Preferably, the protein activity is a binding activity of MCM8 protein to TRIM21 protein or a binding activity of MCM8 protein to mitochondrial LC3 protein.

Preferably, the substance for improving a gene expression or protein expression or protein activity of MCM8 is selected from the group consisting of a micromolecular drug, a polysaccharide drug, a nucleic acid drug, and a polypeptide or protein drug; more preferably, the substance is one or more of a nucleic acid drug containing an MCM8 expression promoter or enhancer, a nucleic acid drug containing the full-length mRNA sequence of MCM8 (e.g., the sequence as shown in SEQ ID NO: 1 and other mRNA isoform sequences of the gene, and a DNA sequence in which U in the mRNA is replaced by T, or a complementary sequence thereto), a nucleic acid drug having any truncated sequence of MCM8 mRNA containing at least a nucleotide sequence shown in SEQ ID NO: 3 (e.g., SEQ ID NO: 5, SEQ ID NO: 6, and a DNA sequence in which U in the mRNA is replaced by T, or a complementary sequence thereto), a polypeptide or protein with the amino acid sequence having any truncated (e.g., SEQ ID NO: 7, SEQ ID NO: 8) or full-length sequence of MCM8 protein (e.g., the sequence as shown in SEQ ID NO: 2 and other amino acid isoform sequences of the protein) containing at least RSFTAL (SEQ ID NO: 4), and a molecular glue capable of binding MCM8 protein to mitochondrial LC3 protein.

Preferably, the drug may further comprise one of or any combination of active ingredients, including: a drug for reducing mitochondrial DNA aggregation in cytoplasm, a drug for inhibiting cGAS-STING signaling pathway activation, a drug for inhibiting IFN-I expression or inhibiting IFN-I binding to a receptor thereof, and other active drugs capable of preventing or treating the disease.

Preferably, the IFN-I is IFN-α and/or IFN-β, and the drug for inhibiting IFN-I expression is a neutralizing antibody against IFN-I, a blocking antibody against IFN-I receptor, or a drug stimulating a subject to produce an antibody against IFN-I.

Preferably, the antibody is a humanized antibody or a human antibody, and the subtype may be selected from the group consisting of IgG1, IgG2, IgG3 or IgG4 subtype.

More preferably, the antibody is one or more of Anifrolumab, Faralimomab, MEDI-545, Rontalizumab, Sifalimumab, AGS-009, IFNα Kinoid, NI-0101, and 1-401.

Preferably, the drug is prepared into a dose form suitable for adults or children, preferably a dose form suitable for children, and the dose forms suitable for children include granules, syrups, chewable tablets, dispersible tablets, suspensions, effervescent tablets, drops, enteric-coated agents, enemas, orally disintegrating tablets, solutions, electuaries and suppositories; and/or a dose form suitable for gastrointestinal administration, transdermal administration, ocular administration, nasal administration or pulmonary administration.

### Beneficial Effects of the Disclosure

In the present disclosure, it is discovered for the first time that MCM8 gene or protein is a target related to the diagnosis, prevention and/or treatment of vasculitis disease such as Kawasaki disease, systemic lupus erythematosus, cardiac vasculitis, pulmonary vasculitis, or vasculitis with abnormal blood composition, pneumonia or severe pneumonia, nephritis, fatty liver, cataract and other diseases, and a target for animal model preparation.

In the present disclosure, it is further discovered that MCM8 gene or protein is a related pathway or target that is involved in the occurrence and development of the above diseases and plays a key role. The pathogenesis and prevention or treatment methods for related diseases are fully expounded, including:
Under-expressed or knockdown or knockout MCM8 is found to be capable of inhibiting mitophagy (including the formation of mitophagosomes) and inhibiting the repair or timely elimination of damaged mitochondria, thus causing more damaged mitochondrial DNA to accumulate in cytoplasm, thereby activating cGAS-STING-IFN-I signaling pathway to produce a large amount of IFN-Is and promote the occurrence and development of many diseases;

Whereas, when MCM8 and the cGAS-STING-IFN-I signaling pathway function normally, under unfavorable factors such as oxidative stress, signal molecules (e.g., NO) signal and then stimulate TRIM21 to bind with MCM8, whereupon intracellular MCM8 is ubiquitinated and then leaves the nucleus, after which the functional LIR-motif sequence (RSFTAL, SEQ ID NO: 4) on MCM8 binds to mitochondrial LC3 to initiate the formation of mitophagosomes, which can eliminate or repair damaged DNAs, inhibiting the aggregation of damaged mitochondrial DNA in cytoplasm, and in turn also inhibiting the activation of cGAS-STING-IFN-I pathway and the occurrence and development of diseases;

Furthermore, the diseases described in the present disclosure (including vasculitis diseases such as Kawasaki disease, cardiac vasculitis, pulmonary vasculitis, systemic lupus erythematosus, and vasculitis with abnormal blood composition, inflammations associated with increased IFN-I, diseases associated with dysfunctional mitophagy, and diseases associated with abnormal activation of cGAS-STING-IFN-I signaling pathway) can be prevented and/or treated by intervention to any one of or any combination of targets in the above signaling pathway, thus providing a new treatment solution to diseases, which is different from the drugs or therapies known in the prior art, and also providing a new technical solution to the prevention and treatment of diseases with reduced drug resistance. For example, when anti-IFNAR is selected to treat diseases including Kawasaki disease and cardiac vasculitis, obvious therapeutic effects are observed.

In particular, the present disclosure has also discovered a diagnostic marker for Kawasaki disease that is closely related to the above mechanism or signaling pathway. The diagnostic marker is MCM8 gene with 826G>C and/or 1094G>A and/or 839C>A and/or 2291C>G mutations, or MCM8 protein with A276P and/or S365N and/or S280C and/or S764A mutations. According to the study of the present disclosure, the diagnostic marker is selected from other MCM8 protein single mutations or linked mutations except A276P mutation present at positions 272-277 of the MCM8 protein sequence of SEQ ID NO: 2, or gene mutations or linked mutations at the corresponding sites (other MCM8 gene single mutations or linked mutations except 826G>C mutation present at positions 816-831 of the MCM8 gene sequence of SEQ ID NO: 1), or other MCM8 gene or protein mutations that also affect LIR function. By means of the above markers, a low-cost rapid diagnosis method is provided for vasculitis diseases such as Kawasaki disease, especially for the diagnosis of Kawasaki disease in East Asian populations.

### BRIEF DESCRIPTION OF DRAWINGS

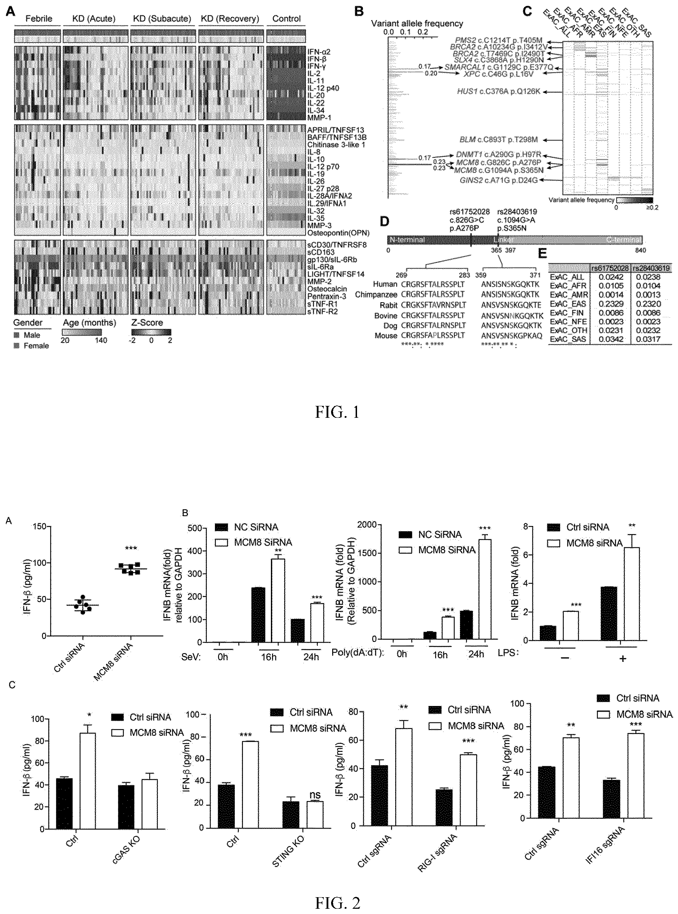

Fig. 1 shows the analysis results of inflammatory factors and whole exon sequencing of Kawasaki disease patients according to the embodiment of the present disclosure, wherein,
   Panel A shows the levels of different cytokines and inflammatory factors in the plasma of Kawasaki disease patients;
   Panel B lists an overview of the mutation frequency of each SNP (single nucleotide polymorphism) carried by Kawasaki disease patients in different human species;
   Panel C lists the mutation frequency of each SNP carried by Kawasaki disease patients in East Asian populations;
   Panel D shows mutation sites of MCM8 and the conservation among different species according to the embodiment of the present disclosure; and
   Panel E shows the specific frequency of the mutation of MCM8 in different human species.
Fig. 2 shows the result that the production of IFN-I mediated by cGAS-STING pathway is promoted upon MCM8 knockdown according to the embodiment of the present disclosure, wherein,
   Panel A shows the result of IFN-β release detected by ELISA after knocking down MCM8 in THP-1 cells with siRNA;
   Panel B shows the results of the expression of IFN-β detected by Q-PCR of THP-1 cells after knocking down MCM8 and stimulated by SeV virus, poly(dA:dT) and LPS bacterial toxin; and
   Panel C shows the release of IFN-β detected by ELISA after knocking down MCM8 in THP-1 cells in which cGAS, STING, RIG-I, or IFI16 has been knocked out respectively.

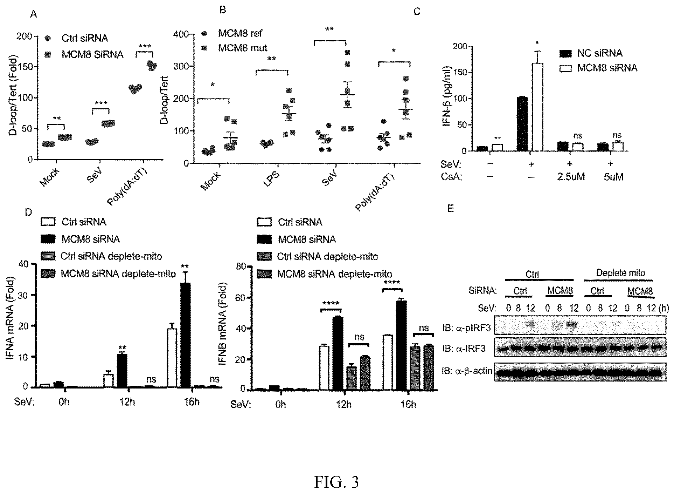

Fig. 3 shows that the release of mitochondrial DNA is promoted by MCM8 knockdown according to the embodiment of the present disclosure, wherein,
   Panel A shows the source of DNA detected by Q-PCR, wherein the DNA is extracted by nuclear-cytoplasmic separation from the cytoplasm of THP-1 cells which are knocked down MCM8 and then stimulated or not stimulated with SeV or poly(dA:dT), respectively;
   Panel B shows the source of DNA detected by Q-PCR, wherein the DNA is extracted by nuclear-cytoplasmic separation from the cytoplasm of peripheral blood mononuclear cells (PBMCs) from Kawasaki disease patients which are grouped according to the genotype of MCM8 (normal group ref and mutant group mut), and then stimulated or not stimulated with LPS, SeV or poly(dA:dT), respectively;
   Panel C shows the release of IFN-α from the THP-1 cells detected by ELISA, wherein the THP-1 cells are THP-1 cells knocked down MCM8, stimulated with SeV, and treated with cyclosporine A (CsA), an inhibitor of mitochondrial permeability transition pores (MTP), or the solvent DMSO, respectively;
   Panel D shows the expressions of IFNA and IFNB of the normal THP-1 or ρ0 THP-1 cells detected by Q-PCR after knocked down MCM8 and then stimulated with SeV, wherein the ρ0 THP-1 cells are constructed by treating THP-1 cells with EtBr to obtain mitochondrion-free ρ0 cells; and
   Panel E shows the levels of pIRF3 of the normal THP-1 or ρ0 THP-1 cells detected by western blot after knocked down MCM8 and then stimulated with SeV, wherein the ρ0 THP-1 cells are constructed by treating THP-1 cells with EtBr to obtain mitochondrion-free ρ0 cells.

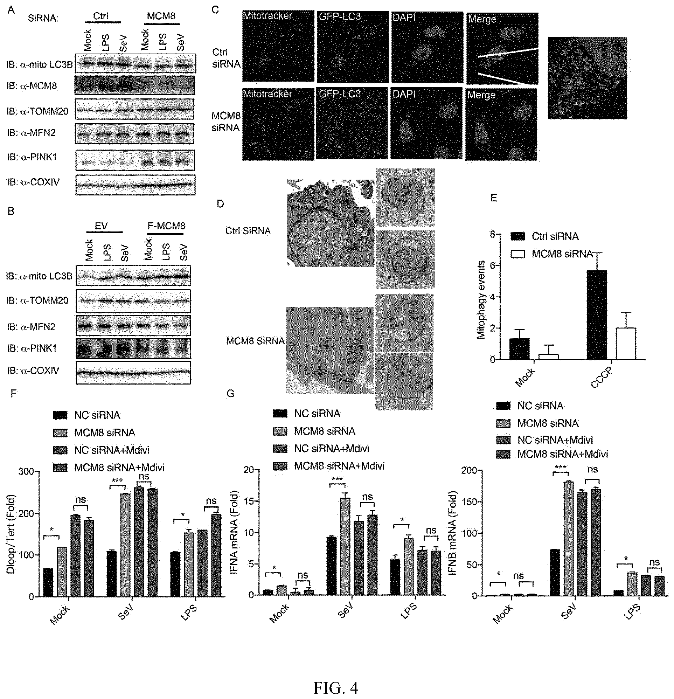

Fig. 4 shows the results that the formation of mitophagosomes is inhibited by MCM8 knockdown according to the embodiment of the present disclosure, wherein,
   Panel A shows the expressions of mitochondrial LC3 and other mitochondrial (TOM20, MFN2, PINK1, and COXIV) proteins of the THP-1 cells detected by western blot after knocked down MCM8 and then stimulated or not stimulated by LPS or SeV, respectively;
   Panel B shows the expressions of mitochondrial LC3 and other mitochondrial (TOM20, MFN2, PINK1, and COXIV) proteins of the Hela cells detected by western blot after knocked down MCM8 and then stimulated or not stimulated by LPS or SeV, respectively;
   Panel C shows the detected co-localization of LC3 and mitochondria in Hela cells which are knocked down MCM8 and stimulated with LPS;
   Panel D shows the number of mitophagies detected by immunoelectron microscopy in THP-1 cells which are knocked down MCM8;
   Panel E shows the results of statistical analysis on the results of immunoelectron microscopy according to Panel D;
   Panel F shows the content of mitochondrial DNA extracted form the cytoplasm of THP-1 cells detected by Q-PCR after knocked down MCM8, stimulated or not stimulated with SeV and LPS, respectively, and treated or not treated with the mitophagy inhibitor Mdivi; and
   Panel G shows the expressions of IFNA and IFNB in THP-1 cells detected by Q-PCR after knocked down MCM8, stimulated or not stimulated with SeV and LPS, respectively, and treated or not treated with the mitophagy inhibitor Mdivi.

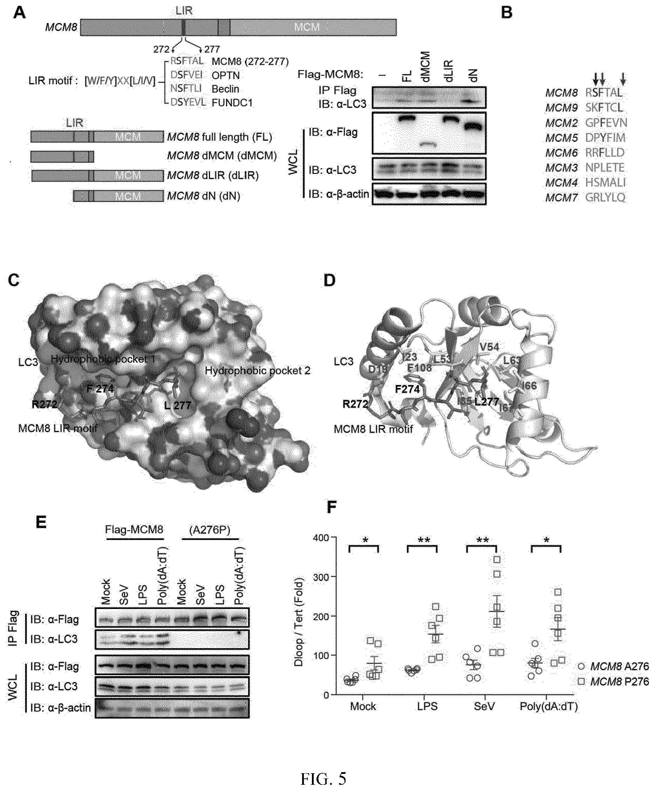

Fig. 5 shows the results of MCM8 acting as mitophagy receptor according to the embodiment of the present disclosure, wherein,
   Panel A shows the detected results of binding to LC3 of various truncated forms of MCM8 constructed with or without LIR motif sequence;
   Panel B shows whether different members of the MCM family have a LIR motif;
   Panel C is a diagram simulating the interaction between LC3 and the tertiary structure of MCM8 LIR motif;
   Panel D is a diagram simulating the interaction between LC3 and the secondary structure of MCM8 LIR motif;
   Panel E shows the results of the interaction between the normal MCM8 or MCM8 with the point mutation A276P and LC3; and
   Panel F shows the detected content of mitochondrial DNA in cytoplasm when human PBMCs with normal A276 site and mutant P276 site are stimulated or not stimulated with LPS, SeV and poly(dA:dT), respectively.

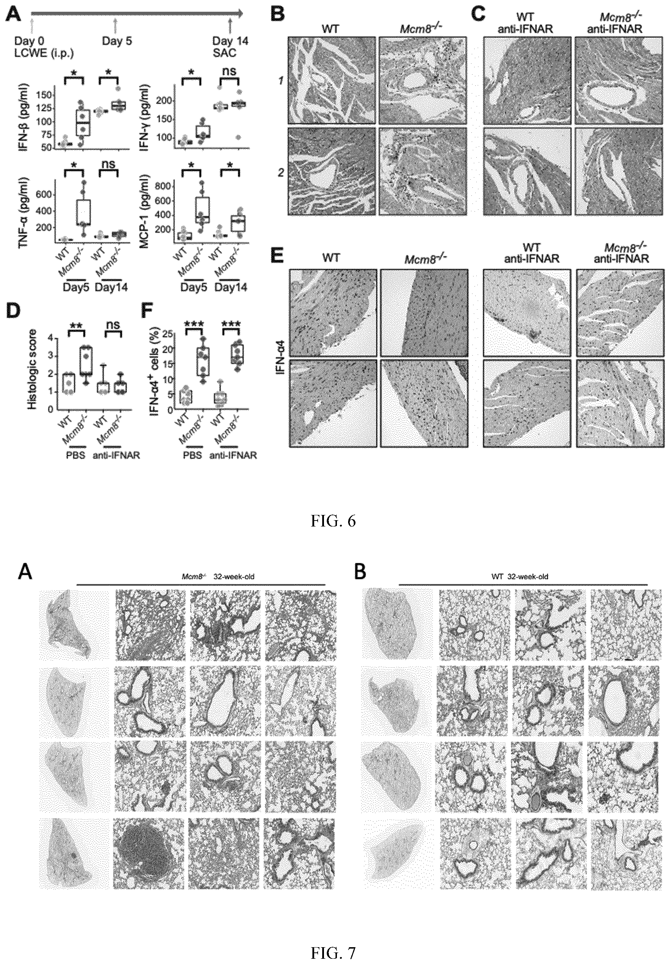

Fig. 6 is a diagram showing a mouse model in which MCM8-knockout promotes the development of Kawasaki disease and the results of the effectiveness of anti-IFNAR treatment according to the embodiment of the present disclosure. In the figure,
   Panel A shows the release of cytokines in the serum of normal and MCM8-knockout LCWE Kawasaki disease mice models at Day 5 (acute phase) and Day 14 (subacute phase);
   Panel B is a schematic diagram showing HE staining of cardiovascular inflammation in mice models at 14 days;
   Panel C shows the HE staining results on the heart tissues of the mice models at 14 days which are subjected to anti-IFNAR treatment;
   Panel D shows the clinical score statistics according to the HE staining results;
   Panel E shows the expression of IFN-α in cardiac sections detected by IHC staining; and
   Panel F is a graph showing the quantitative analysis of IFN-α according to the IHC staining results.
Fig. 7 is a diagram showing pulmonary vasculitis caused by MCM8 deletion, wherein,
   Panel A shows the HE pathological panorama and enlargement of pathological sites of the lungs in MCM8-/- mice; and
   Panel B shows the HE pathological panorama and local enlargement of the lungs in WT mice.
Fig. 8 is a diagram showing that MCM8 deletion aggravates lung inflammation, wherein,
   Panel A is a graphical representation of the method for modeling a COVID-19 infection model of mice;
   Panel B is lung photos of COVID-19 model WT, Mcm8+/- and Mcm8-/- mice;
   Panel C is panoramic and enlarged views of HE pathological sections of the lungs of COVID-19 model WT, Mcm8+/- and Mcm8-/- mice;
   Panel D is enlarged views of HE pathological sites in the lungs of COVID-19 model WT, Mcm8+/- and Mcm8-/- mice;
   Panel E shows edema and necrotic cellular debris in the lungs of MCM8-knockout mice;
   Panel F shows the panorama of the HE staining results of the hearts of MCM8 gene-knockout mice; and
   Panel G shows the enlargement of the HE staining results of the lesion sites of the hearts of MCM8 gene-knockout mice.

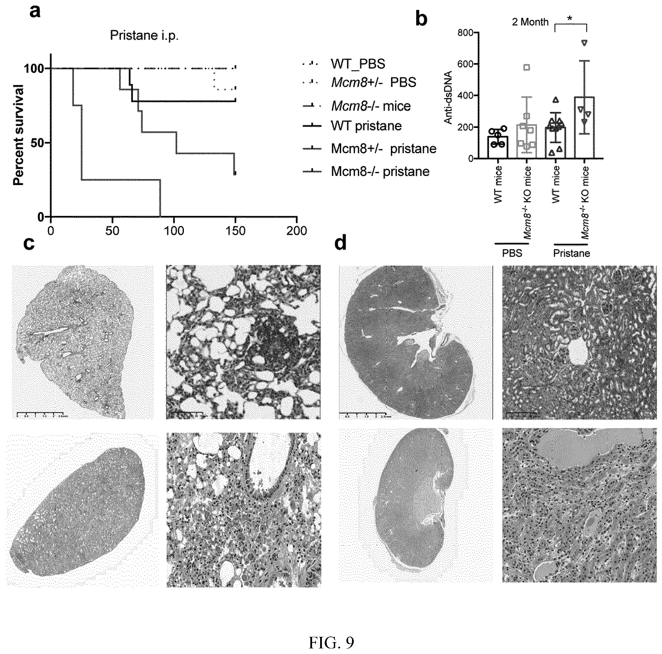

Fig. 9 is a diagram showing the phenotype of nephritis and SLE in mice aggravated by MCM8 deletion, wherein,
   Panel a shows a survival curve of lupus nephritis model mice;
   Panel b shows the concentration of anti-dsDNA antibody in the serum of lupus nephritis model mice;
   Panel c shows the HE pathological sections of the lung and the enlargement of the lesion sites in the mice 4 months after pristane injection; and
   Panel d shows the HE pathological sections of the kidney and the enlargement of the lesion sites in the mice 4 months after pristane injection.

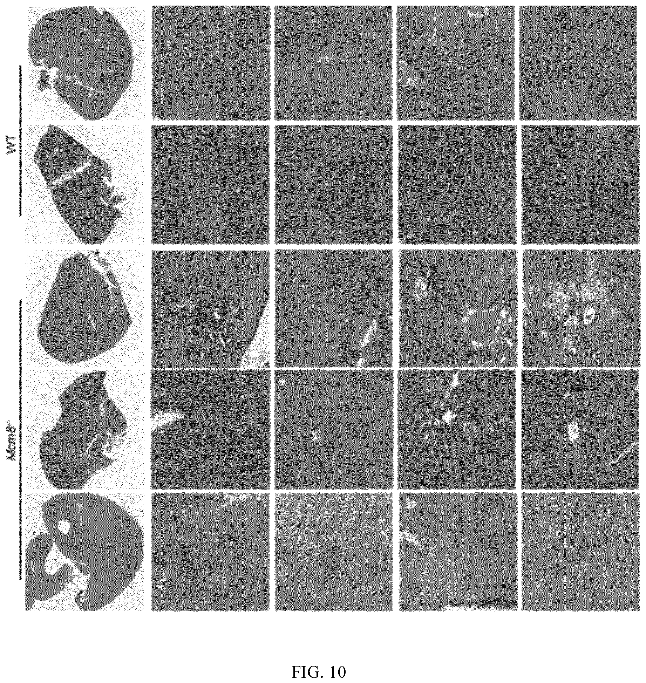

Fig. 10 is an enlarged view showing the HE pathological sections of the lesion sites of steatohepatitis promoted by MCM8 deletion.

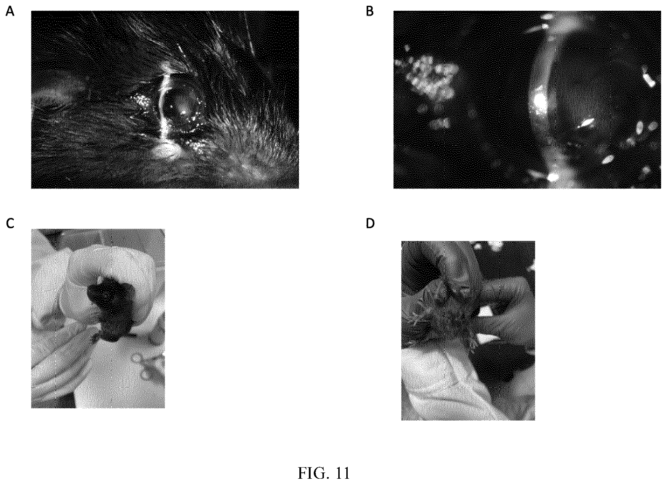

Fig. 11 is images showing corneal lesions in mice caused by MCM8 knockout, wherein,
   Panel A is a graphical representation of ocular surface corneal whitening in MCM8-knockout mice;
   Panel B is an enlarged view of ocular surface corneal whitening in MCM8-knockout mice;
   Panel C is a graphical representation of whitening in the left eye of MCM8-knockout mice; and
   Panel D is a graphical representation of MCM8-knockout mice with no eye on the right side.

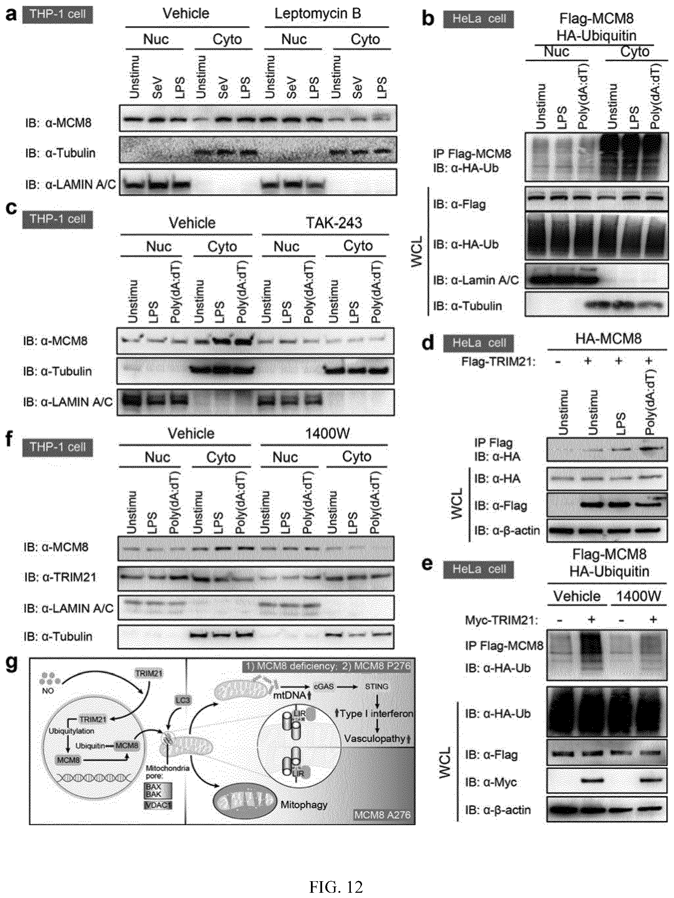

Fig. 12 is a graphical representation of NO promoting TRIM21-mediated MCM8 ubiquitination and nuclear export, wherein,
   Panel a shows that the nuclear export of MCM8 is inhibited by leptomycin B upon stimulation with SeV and LPS;
   Panel b shows that after nuclear-cytoplasmic separation, the ubiquitination level of MCM8 in cytoplasm is significantly higher than that in the nucleus;
   Panel c shows that the nuclear export of MCM8 is inhibited after adding TAK-243, a ubiquitin ligase inhibitor;
   Panel d shows that in HeLa cells, MCM8 is proved able to bind TRIM21 and the binding increases upon stimulation with LPS and poly(dA:dT);
   Panel e shows that TRIM21-mediated MCM8 ubiquitination can be inhibited by NO scavenger 1400W;
   Panel f shows that the nuclear export of MCM8 is inhibited upon addition of NO scavenger 1400W; and
   Panel g is a schematic diagram of MCM8 pathway.

### DETAILED DESCRIPTION

Reference will now be made in detail to embodiments of the present disclosure, one or more examples of which are described hereinafter. Each example is provided as an illustration of, rather than as a limitation to, the present disclosure. In fact, it is obvious to those skilled in the art that various modifications and variations can be made to the present disclosure without departing from the scope or spirit of the present disclosure. For example, features illustrated or described as part of one embodiment can be applied in another embodiment to produce a further embodiment.

Therefore, it is intended that the present disclosure covers such modifications and variations that fall within the scope of the appended claims and the equivalent scopes thereto. The other elements, features and aspects of the present disclosure are disclosed in or become obvious from the following detailed description. It should be understood by those skilled in the art that the description of exemplary embodiments is not intended to limit the broader aspects of the present disclosure.

The present disclosure relates to the use of a reagent for quantitatively detecting a gene expression or protein expression or protein activity of MCM8 in the preparation of a product for the diagnosis of a disease, wherein the disease is a disease caused by dysfunctional mitophagy or a disease caused by abnormal activation of cGAS-STING-IFN-I signaling pathway; wherein if the gene expression or protein expression or protein activity of MCM8 is lower than a reference level, the disease is diagnosed; and the reference level represents the gene expression level or protein expression level or protein activity level of MCM8 in a subject in the same range of age who does not have the disease.

It should be understood by those skilled in the art that reference to "gene sequence" in the present disclosure actually includes any one or two of the complementary double strands, which can be reversely deduced into the corresponding protein sequence. For convenience, in the description and claims, although only one strand is given in most cases, the other strand complementary thereto as well as the corresponding protein sequence has also been disclosed. For example, reference to the mRNA sequence of MCM8 gene actually includes this sequence and the sequence complementary thereto, as well as the sequence of the corresponding translated protein. For example, reference to SEQ ID NO: 1 actually includes the nucleotide sequence complementary thereto and also the corresponding translated amino acid sequence. Those skilled in the art can also understand that one strand can be used to detect the other strand, and vice versa; the gene sequence in the present disclosure comprises mRNA form or cDNA form, and the disclosure of one means that the other is also disclosed. For example, reference to the mRNA sequence of MCM8 gene actually also includes the corresponding cDNA sequence, which is also equivalent to the disclosure and utilization of the sequence in which U in the mRNA sequence is replaced by T and also equivalent to the disclosure or utilization of the sequence complementary thereto. In addition, in order to detect a gene mutation as mentioned above, detection primers and/or probes that can amplify the whole gene can be designed, or detection primers and/or probes may also be designed before and after the position of the mutation.

The term "mitophagy" refers to autophagy that selectively isolates and degrades damaged or incomplete mitochondria, which is involved in maintaining the integrity of mitochondrial network functions and the cell homeostasis. In other words, when mitophagy functions normally or mitophagosomes can be normally initiated and/or formed, damaged DNA can be eliminated or repaired; otherwise, the damaged DNA cannot be repaired, in which case the cytoplasmic DNA aggregation as described in the embodiment of the present disclosure occurs, cGAS-STING-IFN-I signaling pathway is activated, and IFN-I is generated, which induces the occurrence and development of various conditions such as inflammations and cancers. At present, most of the known diseases associated with mitophagy are related to neurological diseases, cardiovascular diseases and cancers. Therefore, intervention in mitophagy has a therapeutic potential for human diseases associated with mitochondrial dysfunction. At present, it is known that small molecular synthetic or natural compounds such as urea A are proved helpful in phagocytosis and mitosis.

Herein, the term "cGAS-STING-IFN-I signal pathway" or "cGAS-STING-IFN-I signaling pathway" refers to a process of recognition of DNA by DNA receptor cGAS and signal transduction to STING to activate Interferon-I (IFN-I). The IFN-I is Interferon α (IFN-α) and/or Interferon β (IFN-β).

The diseases caused by abnormal activation of cGAS-STING-IFN-I signaling pathway include autoimmune diseases, inflammatory diseases, aging-related diseases, cancers etc.

In addition, reference to "MCM8-cGAS-STING-IFN-I signal pathway" or "MCM8-cGAS-STING-IFN-I signaling pathway" hereinafter means that by controlling the expression upstream or downstream of MCM8, for example, by controlling the binding of TRIM21 to MCM8, ubiquitinating MCM8 (and/or accompanied by phosphorylation), and by promoting the binding of MCM8 to mitochondrial LC3, the cGAS-STING-IFN-I signaling pathway is regulated.

The terms "ubiquitination" and "phosphorylation" are common protein modifications in eukaryotic cells. Ubiquitin plays an important targeting role in proteasome degradation pathway and extracellular signals strictly regulate the ubiquitination of the protein of interest. In many cases, this regulation is also accompanied by protein-dependent phosphorylation.

Interferon (IFN) is a broad-spectrum antiviral agent, which can inhibit virus replication and can also enhance the viability of natural killer cells (NK cells), macrophages and T lymphocytes, thus playing an immunomodulatory role and enhancing antiviral ability. Interferons are a group of multifunctional active proteins (mainly glycoproteins) and are cytokines produced by monocytes and lymphocytes. Interferon has a broad spectrum of antiviral activities on the same kind of cells, affecting cell growth and various biological activities such as differentiation and immune function regulation. At present, interferons are generally divided into three types, of which type I includes IFN-α and IFN-β. IFN-I has the functions of inhibiting virus replication, resisting parasites, inhibiting the proliferation of various cells, stimulating the killing activity of immune cells, participating in immune regulations, resisting tumors, etc. IFN-ω, which belongs to the IFN-α family, is slightly different in structure and size but quite different in antigenicity from other IFN-α.

Furthermore, the subtypes of IFN-α is at least one human Interferon-α (IFN-α) subtype selected from IFN-ω, IFN-αA, IFN-αB2, IFN-αC, IFN-αF, IFN-αG, IFN-aH2, IFN-αI, IFN-αJ1, IFN-αK, IFN-αWA and IFN-α4a.

In some embodiments, the reagent is used to inhibit the expression of IFN-I or inhibit the binding of IFN-I to its receptor.

In one embodiment, the product comprises a kit, a diagnostic reagent, a detection reagent, a gene chip or a protein chip, wherein the kit may also comprise the diagnostic reagent, the detection reagent, the gene chip or the protein chip.

In one embodiment, the protein activity is a binding activity of MCM8 protein to TRIM21 protein or a binding activity of MCM8 protein to mitochondrial LC3 protein.

"TRIM21" is a human anti-E3 ubiquitin ligase, and TRIM21 protein is widely expressed in various types of cells, especially in some immune cells such as T cells, macrophages, and dendritic cells. TRIM21 was first discovered as an autoantigen, also known as Ro52/SS-A, which often causes autoimmune diseases such as Sjogren's syndrome, systemic lupus erythematosus and systemic sclerosis, and plays an important role in the regulation of innate immunity and adaptive immunity. TRIM21 protein is the only intracytoplasmic IgG receptor found in mammals at present. In addition, TRIM21 protein also regulates the secretion of various cytokines in innate immunity.

Exemplary detection methods for the binding activity comprises detecting the ubiquitination level of MCM8 protein when detecting the binding activity of MCM8 protein to TRIM21 protein; and detecting the binding activity of LIR motif sequence with amino acid sequence RSFTAL in MCM8 protein to mitochondrial LC3 protein when detecting the binding activity of MCM8 protein to mitochondrial LC3 protein.

In one embodiment, the reagent for quantitatively detecting a gene expression or protein expression or protein activity of MCM8 in the preparation of a product for the diagnosis of a disease is configured for performing one or more of the detection methods selected from the group consisting of: various PCR related methods, including polymerase chain reaction, micro digital polymerase chain reaction, fluorescence polymerase chain reaction, and loop-mediated isothermal amplification reaction; enzyme-linked immunosorbent assay (ELISA); and nucleotide or amino acid sequence sequencing; denaturing gradient gel electrophoresis, nucleic acid typing chip detection, high performance liquid chromatography, in-situ hybridization, biological mass spectrometry, high-resolution melting curve analysis, single-strand conformational isomerism polymorphism analysis, or probe amplification-refractory mutation system analysis.

In one embodiment, the product contains a primer and/or detection probe for detecting MCM8 gene or control gene; and/or further contains a sample treatment reagent, including but not limited to a sample lysis reagent, a sample purification reagent and/or a sample nucleic acid extraction reagent; and/or further contains one or more of DNA extraction reagent, dNTP, DNA polymerase, double-strand specific fluorescent dye, and water.

Herein, the "control gene" refers to a class of genes which are stably expressed in all cells and have the products which are necessary to maintain the basic life activities of cells, e.g., tubulin gene, glycolytic enzyme gene and ribosomal protein gene.

In one embodiment, the product is used for a detection specimen or test sample comprising one or more of blood, body fluid, urine, tissue and cell samples from a subject to be tested; the subject to be tested is an adult or a child, preferably a child.

In the present disclosure, the term "knockdown" means that the expression of the target gene of MCM8 is reduced by using miRNA, siRNA, antisense nucleotide, etc.

In addition, as mentioned above, the reagent can act on the DNA level, mRNA level or protein level. When the reagent is an inhibitory reagent (for example, a reagent for inhibiting cGAS-STING, especially IFN-I), the protein level inhibitor may be a neutralizing antibody against the target, or a blocking antibody against the receptor or upstream molecule, or a substance that may block the activity, such as a compound.

RNA level inhibitors are, for example, miRNA, siRNA, antisense nucleotide, etc.

DNA-level inhibitors are, for example, various reagents that can knock out the full-length or partial fragments of the target gene; preferably CRISPR system.

In some embodiments, the reagent is used to reduce the aggregation of mitochondrial DNA in cytoplasm. In addition, the present disclosure further relates to the use of MCM8 gene-knockout mice in the preparation of an animal model of disease, wherein the disease is a disease caused by dysfunctional mitophagy or a disease caused by abnormal activation of GAS-STING-IFN-I signaling pathway.

The disclosure further relates to the use of a substance for improving a gene expression or protein expression or protein activity of MCM8 in the preparation of a drug for the prevention and/or treatment of a disease, wherein the disease is a disease caused by dysfunctional mitophagy or a disease caused by abnormal activation of cGAS-STING-IFN-I signaling pathway.

In one embodiment, the protein activity is a binding activity of MCM8 protein to TRIM21 protein or a binding activity of MCM8 protein to mitochondrial LC3 protein.

The substance for improving a gene expression or protein expression or protein activity of MCM8 may be selected from the group consisting of a micromolecular drug, a polysaccharide drug, a nucleic acid drug, and a polypeptide or protein drug; more preferably the substance is one or more of a nucleic acid drug containing an MCM8 expression promoter or enhancer, a nucleic acid drug having any truncated (e.g., SEQ ID NO: 5 and SEQ ID NO: 6) or full-length sequence of MCM8 mRNA (e.g., SEQ ID NO: 1) containing at least the nucleotide sequence 5'-AGGUCAUUUACUGCUCUC-3' (shown in SEQ ID NO: 3) and a DNA sequence in which U in the mRNA is replaced by T, or a complementary sequence thereto, a polypeptide or protein having any truncated (e.g., SEQ ID NO: 7 and SEQ ID NO: 8) or full-length sequence of MCM8 protein (e.g., SEQ ID NO: 2) containing at least the amino acid sequence RSFTAL (SEQ ID NO: 4), and a molecular glue capable of binding MCM8 protein to mitochondrial LC3 protein. The "nucleic acid drug containing the full-length mRNA sequence of MCM8" and the "polypeptide or protein having any truncated or full-length sequence of MCM8 protein containing at least RSFTAL", etc. are exogenously expressed mRNA, proteins or functional fragments of MCM8, or components capable of loading or expressing the above substances, optionally for example, expression vectors (such as plasmid vectors), liposomes, lentiviruses and adenoviruses.

In addition, the present disclosure further relates to a pharmaceutical composition for the prevention and/or treatment of a disease, wherein the disease is a disease caused by dysfunctional mitophagy or a disease caused by abnormal activation of cGAS-STING-IFN-I signaling pathway, and the pharmaceutical composition contains a combination of a) and c) or a combination of a), b) and c):
a) one of or any combination of a drug for improving the gene expression of MCM8, a drug for improving the protein expression of MCM8 (e.g., exogenously expressed mRNA or proteins or functional fragments of MCM8, or components capable of loading or expressing the above substances, optionally for example, expression vectors, liposomes and lentiviruses), a drug for improving the binding of MCM8 protein to TRIM21 protein, a drug for improving the ubiquitination or phosphorylation modification of MCM8 in nucleus, and a drug for improving the binding of MCM8 protein to mitochondrial LC3 protein,
   wherein a) is selected from the group consisting of a micromolecular drug, a polysaccharide drug, a nucleic acid drug, and a polypeptide or protein drug; more preferably, a) is a nucleic acid drug containing an MCM8 expression promoter or enhancer, a nucleic acid drug having any truncated (e.g., SEQ ID NO: 5 and SEQ ID NO: 6) or full-length sequence of MCM8 mRNA (e.g., SEQ ID NO: 1) containing at least the nucleotide sequence 5'-AGGUCAUUUACUGCUCUC-3' (shown in SEQ ID NO: 3) and a DNA sequence in which U in the mRNA is replaced by T, or a complementary sequence thereto), a polypeptide or protein having any truncated (e.g., SEQ ID NO: 7 and SEQ ID NO: 8) or full-length sequence of MCM8 protein (e.g., SEQ ID NO: 2) containing at least the amino acid sequence RSFTAL (SEQ ID NO: 4), and a molecular glue capable of binding MCM8 protein to mitochondrial LC3 protein;
b) one of or any combination of a drug for improving mitophagosome formation (e.g., urea A), a drug for reducing mitochondrial DNA aggregation in cytoplasm, a drug for inhibiting cGAS-STING signaling pathway activation, a drug for inhibiting IFN-I expression, and other active drugs capable of preventing or treating the disease,
   wherein a drug for inhibiting IFN-I expression is preferred, the IFN-I is IFN-α and/or IFN-β, the drug for inhibiting IFN-I expression is a neutralizing antibody against IFN-I, a blocking antibody against IFN-I receptor, or a drug stimulating a subject to produce an antibody against IFN-I, more preferably, the antibody is one or more of Anifrolumab, Faralimomab, MEDI-545, Rontalizumab, Sifalimumab, AGS-009, IFNα Kinoid, NI-0101, and 1-401; and
c) a delivery vector for a nucleic acid, polypeptide or protein (e.g., expression vector, liposome, or lentivirus), and/or a pharmaceutically acceptable carrier or adjuvant.

In some embodiments, the antibody is specific and preferably has an affinity of 108 1/mol or more preferably 109 1/mol to the target.

According to the present disclosure, under a normal mechanism, when cells are under oxidative stress, they produce NO signal molecules, and the NO promotes the translocation of TRIM21 into the nucleus, after which TRIM21 binds to MCM8 and ubiquitinates MCM8. The ubiquitinated MCM8 leaves the nucleus, locates into the mitochondria and recruits LC3 via the LIR-motif to initiate mitophagy, which eliminates damaged mitochondria. MCM8, when under-expressed or mutated, the mitophagy and timely clearance of damaged mitochondria may be inhibited, thereby causing more damaged mitochondrial DNAs to accumulate in cytoplasm, thus activating cGAS-STING-IFN-I signaling pathway to produce more IFN-Is and promote the occurrence and development of multiple diseases. The diseases mentioned in the present disclosure (including Kawasaki disease, vasculitis, inflammations associated with increased IFN-I, diseases associated with dysfunctional mitophagy, and diseases associated with abnormal activation of cGAS-STING-IFN-I signaling pathway, etc.) can be prevented and/or treated by intervention to any targets in the above signal pathway.

As used herein, the term "target" may include, but is not only limited to, target genes, target mRNAs, target proteins, etc., and the reagent can function by additionally administering the target or inhibiting the target. As is obvious to those skilled in the art, the target mRNA should not be interpreted as limited to the specific mRNA sequences obtained by direct transcription of the above genes. For example, when the reagent is siRNA, it can bind with partial sequences of target mRNA, etc. to block the function of the whole target mRNA. Similarly, the protein or polypeptide used as the target in the present disclosure is expected to include naturally occurring fragments of the protein, especially the functional fragments. In addition, or in the alternative, the target protein may carry post-translational modifications. Non-limiting examples of post-translational modifications are ubiquitination, glycosylation, acylation and/or phosphorylation.

The term "antibody" includes polyclonal antibodies and monoclonal antibodies, and the term "antibody fragment" includes antigen compound binding fragments of such antibodies, including Fab, F(ab')2, Fd, Fv, scFv, bispecific antibodies, and antibody minimal recognition units, as well as single-chain derivatives of these antibodies and fragments, such as scFv-Fc. The type of the antibody may be selected from the group consisting of IgG1, IgG2, IgG3, IgG4, IgA, IgM, IgE, and IgD. In addition, the term "antibody" includes naturally occurring antibodies as well as non-naturally occurring antibodies, including, for example, chimeric, bifunctional, humanized, and human antibodies, and related synthetic isoforms. The term "antibody" can be used interchangeably with "immunoglobulin". In some preferred embodiments, the reagent is a humanized antibody or a human antibody.

The "humanized antibody" refers to an antibody in which the antigen binding site is derived from a non-human species and the variable region framework is derived from a human immunoglobulin sequence. The humanized antibody may contain substitutions in the framework region, such that the framework may not be an exact copy of the expressed human immunoglobulin sequence or germline gene sequence.

The "human antibody" refers to an antibody with a heavy chain variable region and a light chain variable region in which both the framework region and the antigen binding site region are derived from sequences of human origin. If the antibody comprises a constant region, the constant region is also derived from a sequence of human origin.

In some embodiments, the humanized antibody or human antibody is IgG1, IgG2, IgG3 or IgG4 subtype.

In some embodiments, the reagent is one or more of Anifrolumab, Faralimomab, MEDI-545, Rontalizumab, Sifalimumab, AGS-009, IFNα Kinoid, NI-0101, and 1-401.

In one embodiment, the disease caused by dysfunctional mitophagy and/or the disease caused by abnormal activation of cGAS-STING-IFN-I signaling pathway are selected from the group consisting of autoimmune diseases, inflammatory diseases, neurological conditions, aging-related diseases, or cancers.

In one embodiment, the autoimmune disease is selected from the group consisting of systemic lupus erythematosus, systemic pulmonary fibrosis (SSc), myositis, Sjogren's syndrome, Aicardi syndrome, Singleton-Merten syndrome, cutaneous vasculitis, rheumatoid arthritis (RA), multiple sclerosis (MS) and STING-related vasculitis.

In one embodiment, the inflammatory disease is selected from the group consisting of infectious diseases cause by viral, bacterial, or fungal infection, silvery tinea, psoriasis, inflammatory bowel disease (IBD), cystic fibrosis (CF), herpes, asthma and allergy, sepsis and septic shock, dengue hemorrhagic fever, type I diabetes, endometriosis, prostatitis, uveitis and uterine maturity; preferably, the inflammatory disease is an inflammatory disease mediated by IFN-I.

In one embodiment, the neurological condition is selected from the group consisting of frontotemporal lobar degeneration, Alzheimer's disease, Parkinson's disease, dementia with Lewy bodies, corticobasal degeneration, progressive supranuclear paralysis, Guam Parkinsonism-dementia ALS complex, Huntington's chorea, inclusion body myopathy with early-onset Paget's disease and frontotemporal dementia, inclusion body myositis, myofibrillar myopathy, boxer's dementia, chronic traumatic encephalopathy, Alexander disease, hereditary inclusion body myopathy, amyotrophic lateral sclerosis, congenital myasthenia syndrome, congenital myopathy, cramp-fasciculation syndrome, Duchenne muscular dystrophy, glycogenosis type II, hereditary spastic paraplegia, Isaacs' syndrome, Kearns-Sayre syndrome, Lambert-Eaton myasthenic syndrome, mitochondrial myopathy, muscular dystrophy, myasthenia gravis, myotonic dystrophy, peripheral neuropathy, spinal and bulbar muscular atrophy, spinal muscular dystrophy, Stiff person syndrome, Taylor syndrome and Guillain-Barre syndrome, and amyotrophic lateral sclerosis (ALS).

In one embodiment, the disease caused by aging is selected from the group consisting of cardiovascular diseases, osteoporosis, age-related lung diseases, age-related eye diseases, age-related liver diseases, age-related renal diseases, age-related hearing loss, age-related muscle fatigue or sarcopenia, age-related skin conditions, pancreatic fibrosis, and oral submucosal fibrosis.

In one embodiment, the cancer is selected from the group consisting of lung cancer, gastric cancer, liver cancer, colorectal cancer, melanoma, nephroma, ovarian cancer, prostate cancer, bladder cancer, breast cancer, esophageal cancer, large intestine cancer, nasopharyngeal cancer, brain tumor, cervical cancer, hematologic cancer, bone cancer, lymphoma, and pancreatic cancer.

In one embodiment, the STING-related vasculitis is selected from the group consisting of Kawasaki disease, cardiac vasculitis, and cutaneous vasculitis.

The infectious disease caused by viral, bacterial, or fungal infection is selected from the group consisting of bacterial pneumonia, viral pneumonia including influenza and novel coronavirus pneumonia, bacterial meningitis respiratory conditions, pneumonia caused by respiratory syncytial virus (RSV), and bacterial enteritis.

The inflammatory bowel disease is selected from the group consisting of indeterminate colitis, ulcerative colitis, or Crohn's disease.

The cardiovascular disease is selected from the group consisting of atherosclerosis, angina pectoris, arrhythmia, cardiomyopathy, congestive heart failure, coronary artery disease, carotid artery disease, endocarditis, coronary artery thrombosis, myocardial infarction, hypertension, aortic aneurysm, diastolic dysfunction, hypercholesterolemia, hyperlipidemia, mitral valve prolapse, peripheral vascular disease, cardiac stress resistance, myocardial fibrosis, cerebral aneurysm, high blood pressure and stroke.

The age-related renal disease is selected from the group consisting of renal failure, renal weakness, and renal fibrosis.

The age-related lung disease is selected from the group consisting of pulmonary fibrosis, chronic obstructive pulmonary disease, asthma, cystic fibrosis, emphysema, and bronchiectasis.

The age-related skin condition is selected from the group consisting of eczema, psoriasis, hyperpigmentation, nevus, rash, atopic dermatitis, urticaria, diseases and conditions associated with photosensitivity or photoaging, wrinkles, itching, insensitivity, eczema outbreak, eosinophilic dermatosis, reactive neutrophilic dermatosis, pemphigus, pemphigoid, immune bullous dermatosis, skin fibrous histiocytosis, and skin lymphoma.

The age-related eye disease is an eye disease or condition selected from the group consisting of macular degeneration, glaucoma, cataract, presbyopia, and vision loss.

In a preferred embodiment, the disease caused by dysfunctional mitophagy or the disease caused by abnormal activation of cGAS-STING-IFN-I signaling pathway is selected from the group consisting of vasculitis, pneumonia, nephritis, systemic lupus erythematosus, steatohepatitis and cataract, and more preferably, the vasculitis is preferably Kawasaki disease, systemic lupus erythematosus, cardiac vasculitis, pulmonary vasculitis, or vasculitis with abnormal blood composition.

In the present disclosure, examples of the pharmaceutically acceptable carriers or adjuvants include a binder (e.g. syrup, Arabic gum, gelatin, sorbitol, tragacanth, polyvinylpyrrolidone, etc.), a filler (e.g. lactose, sucrose, starch, calcium phosphate, sorbitol, glycine, etc.), a lubricant (e.g. magnesium stearate, talcum, polyethylene glycol, etc.), a disintegrant (e.g. starch, microcrystalline cellulose, etc.), a wetting agent (e.g. sodium dodecyl sulfate, etc.), a suspending agent (e.g. sorbitol, syrup, methyl cellulose, glucose syrup, gelatin, hydrogenated edible fat, etc.), an emulsifier (lecithin, sorbitol monooleate, Arabic gum, etc.), a non-aqueous carrier (e.g. almond oil, fractionated coconut oil or glycerol, propylene glycol, ethanol and other hydrophobic esters, etc.), a preservative (e.g. methyl or propyl paraben, sorbic acid, etc.), aromatics (e.g. synthetic fragrances, natural fragrances, etc.), a sweetener (e.g. sucrose, stevia, xylitol, etc.), a pH regulator (e.g. sodium bicarbonate, potassium carbonate, etc.), a powder (e.g. pigment, dye, resin, etc.), a thickener (e.g. Arabic gum, methyl cellulose, etc.), an antioxidant (e.g. vitamin C, vitamin E, etc.), etc.

The above drug or pharmaceutical composition of the present disclosure can be prepared into a dose form suitable for adults or children, preferably suitable for children. The dose form for adults can be prepared as tablets, pills, powders, granules, capsules, liquids and solutions, suspensions, emulsions, injections, drops, etc. as long as the reagent can be administered; and the dose form suitable for children optionally comprises granules, syrups, chewable tablets, dispersible tablets, suspensions, effervescent tablets, drops, enteric-coated agents, enemas, orally disintegrating tablets, solutions, electuaries, suppositories, etc. The present disclosure is not intended to limit any administration method for drugs, and the specific administration method can be appropriately selected from the group consisting of oral administration, injection (e.g. subcutaneous, intradermal, intramuscular, intravenous, and intra-arterial), dermal administration, transdermal administration, etc. It is preferably prepared into a dose form suitable for gastrointestinal administration, dermal administration, ophthalmic administration, or pulmonary administration.

In some embodiments, the drug or pharmaceutical composition may also comprise other therapeutic agents for Kawasaki disease.

In some embodiments, the therapeutic agent includes one or more doses of IVIG, aspirin, anti-TNF-α agent, corticosteroid, cyclosporine, IL-1 inhibitor (anakinra and canakinumab), cyclophosphamide, Rituximab, Tocilizumab, Pentoxifylline, plasmapheresis or any combination thereof.

According to yet another aspect, the present disclosure further relates to a method for the treatment, prevention, mitigation and/or alleviation of the above disease, which comprises a step of administering an effective amount of the above reagent or drug to a subject.

The term "effective amount" mentioned in the present disclosure refers to the dose of the component corresponding to the term to achieve treatment, prevention, mitigation and/or alleviation of the disease or condition mentioned in the present disclosure in a subject.

The term "subject" mentioned in the present disclosure may refer to a patient or other animals that receive the reagent or drug according to the present disclosure to treat, prevent, mitigate and/or alleviate the diseases, conditions and symptoms mentioned in the present disclosure. The subject includes warm-blooded animals such as mammals, e.g., primates, and preferably humans. Non-human primates are also individuals. The term individual includes domesticated animals, such as cats and dogs, domestic animals (for example, cattle, horses, pigs, sheep, goats, etc.) and experimental animals (for example, mice, rabbits, rats, gerbils, guinea pigs, etc.).

The present disclosure further relates to a diagnostic marker for vasculitis, wherein the biomarker is MCM8 gene with 826G>C and/or 1094G>A and/or 839C>A and/or 2291C>G mutations, or the mutation is MCM8 protein with A276P and/or S365N and/or S280C and/or S764A mutations; or other MCM8 protein single mutations or linked mutations except A276P mutation present at positions 272-277 of the MCM8 protein sequence of SEQ ID NO: 2, or gene mutations or linked mutations at the corresponding sites (other MCM8 gene single mutations or linked mutations except 826G>C mutation present at positions 816-831 of the MCM8 gene sequence of SEQ ID NO: 1).

The present disclosure will be further illustrated hereinafter with reference to the attached drawings and specific examples, and the examples are not intended to limit the present disclosure by any way. For those skilled in the art, any other changes, modifications, substitutions, combinations, and simplifications made without departing from the spirit and principles of the present disclosure should be equivalent replacements and included in the scope of the present disclosure.

Unless otherwise specified, the reagents, methods and equipment used in the present disclosure are conventional reagents, methods, and equipment in the art. Unless otherwise specified, the reagents and materials used in the following examples are all commercially available.

Unless otherwise specified, immunology, biochemistry, chemistry, molecular biology, microbiology, cell biology, genomics, recombinant DNAs and the like used in the present disclosure are conventional means in the art. See Sambrook, Fritsch and Maniatis, Molecular Cloning:A Laboratory Manual, 2nd edition (1989); Current Protocols in Molecular Biology, edited by F.M. Ausubel et al., (1987); Methods in Enzymology series, Academic Press: PCR 2: A Practical Approach, edited by M.J. MacPherson, B.D. Hames and G.R. Taylor (1995); Antibodies, A Laboratory Manual, edited by Harlow and Lane (1988); and Animal Cell Culture, edited by R.I. Freshney (1987).

### EMBODIMENTS

In Embodiments 1-9, Kawasaki disease is taken as an example to illustrate the regulatory mechanism of MCM8-cGAS-STING-IFN-I signaling pathway on the disease.

### Embodiment 1: IFN-I level abnormally increases in Kawasaki disease patients.

Kawasaki disease is a kind of systemic vasculitis in children with the pathogenesis still unknown. At present, it is generally believed that the disease is induced by infectious factors. Therefore, in order to study whether there are abnormal cytokine levels caused by infection and other factors in Kawasaki disease patients, the inventors collected bloods from 42 Kawasaki disease patients, 30 non-Kawasaki disease patients with fever and 25 healthy controls and detected inflammatory factor indicators in plasma using a 37-factor kit from Bio-Plex Pro according to the steps in the instructions. The results show that except for traditional inflammatory factors, the levels of IFN-I (IFN-α and IFN-β) are abnormally increased and remain high in different phases of the disease (acute phase, subacute phase and rehabilitation phase) (see Panel A, Fig. 1). The results suggest that IFN-I plays an important role in the development of Kawasaki disease and may become a potential target for disease diagnosis, prevention and/or treatment.

### Embodiment 2: MCM8 mutations are found in whole exon sequencing analysis on Kawasaki disease patients.

Kawasaki disease, which highly occurs in East Asian populations, has genetic susceptibility. In order to explore the susceptibility genes of the disease and explore the susceptibility mechanism, blood samples from 130 Kawasaki disease patients (from Guangzhou Women and Children's Medical Center, and the collection had been approved by the ethical review of clinical research of the Guangzhou Women and Children's Medical Center) were first subjected to whole exon sequencing and analyzed by GWAS (Genome Wide Association Study). By focusing on genes with significant differences among populations in mutation frequency and regulatory genes related to chromosome stability, it was found from the results that mutations in chromatin maintenance genes, especially in MCM8 gene, were a susceptibility factor specific for East Asian populations (see Panels B and C, Fig. 1). The two mutations A276P and S365N (with the corresponding gene mutation site information of c.826G>C and c. 1094G>A, see Panels B and C, Fig. 1) in MCM8 detected by the inventors are located in rs61752028 and rs28403619, respectively (see Panel D, Fig. 1). The above mutation sites are linked mutations and are conservative among different species. The frequencies of the two mutations are enriched as high as 28% or more in the collected Kawasaki disease patients, and the frequencies of the two mutations are as high as 23% or more in the East Asian populations, while the frequencies of the mutations in non-East Asian populations are only 0.1-3% (see Panels D and E, Fig. 1).

Besides the previously found A276P and S365N mutations, the inventors found other point mutations related to Kawasaki disease, namely, S280C mutation and S764A mutation, and further conducted cloning and cell experiments, comprising the steps of using 293T cells to separately overexpress 200 ng MCM8, and each of mutants, namely, MCM8 (A276P), MCM8 (S280C), MCM8 (S365N) and MCM8 (S764A); and 24 hours after transfection, stimulating the cells with poly(dA:dT) for 0, 12 and 24 h, collecting RNA, and detecting the mRNA level of IFN-β by Q-PCR. As a result, the IFN-β level of those that overexpressed MCM8 was 0, 100 and 250, respectively at 0 h, 12 h and 24 h, whereas the mutations A276P, S365N and S764A in MCM8 all led to increased IFN-β levels (more than 300 at 24 h), especially for the mutation S365N by which the increase was the most significant (1000 at 24 h, P < 0.001 as compared to the overexpressed MCM8), and the mutation S280C did not lead to the increase of IFN-β level (below 250 at 24 h). Therefore, it can be further considered that the previously found mutations A276P and S365N in MCM8 are both mutation factors that affect the function of MCM8 in regulating IFN-I and lead to Kawasaki disease, while the mutations S280C and S764A are also newly discovered mutations in systemic vasculitis such as Kawasaki disease. These two mutations may be used to diagnose or assist in the diagnosis of Kawasaki disease and systemic vasculitis diseases similar to Kawasaki disease (e.g., systemic lupus erythematosus).

### Embodiment 3: Infected MCM8-knockdown cells produce more IFN-I.

In order to further clarify the influence of the expression of MCM8 on the production of IFN-I, the experiment of Embodiment 3 was carried out.

By knocking down MCM8 with siRNA in THP-1 cell line (a human leukemia cell line, which represents white blood cells, which are a large class of blood cells and are ideal cells model for the study of vasculitis-related diseases, especially vasculitis with abnormal blood composition), a phenomenon of insufficient expression of MCM8 in the body was simulated. The knockdown was specifically conducted as follows: 50 pmol of siRNA was added to 25 µl of OPTI-MEM medium; at the same time, 2 µl of lipofectamine RNAi Max was added to 25 µl of OPTI-MEM medium; and the two were thoroughly mixed and then left to stand at room temperature for 10 minutes, and the transfection complex was added to a 24-well plate plated with 1 x 10⁶ THP-1 cells/ml. After MCM8 in the THP-1 cells was knocked down, the inventors detected the release of IFN-β in the supernatant of THP-1 cells by ELISA.

Since the pathogen of Kawasaki disease was unknown, both viruses and bacteria were possibly the inducement of Kawasaki disease. The inventors simulated viral and bacterial stimulations using SeV (MOI 1), poly(dA:dT) (1 ug/ml) and LPS (200 ng/ml) (the same below), respectively, collected the supernatant, and detected by IFN-specific ELISA from Thermo Fisher Scientific according to the corresponding instructions or by extracting RNA from cells with TRIZOL and detecting with Vazyme reverse transcription kit and QPCR kit according to the instructions. The specific experimental method for RNA extraction and QPCR detection was as follows: RNA extraction: 1 ml of TRIZOL was added to an amount of cells; after violent shaking for 15 s, the cells were incubated at room temperature for 5 minutes to fully lyse the cells; 200 µl of chloroform was added, and after strong Vortex for 15 s, the cell lysate was incubated at room temperature for 2-15 min; the cell lysate was centrifuged at 12,000 x g for 15 minutes, and the aqueous phase was transferred; the aqueous phase was mixed with 500 µl of isopropanol, the mixed liquid was then incubated at room temperature for 5-10 minutes; the mixed liquid was centrifuged at 12,000 x g for 10 minutes and the supernatant was removed; the precipitated RNA was immersed with 1 ml of ethanol, shaken and washed; the washing solution was centrifuged at 7,500 x g for 5 minutes, and excess ethanol was removed; and after natural air drying, 20 µl of DEPC water was added to dissolve RNA.

The results are as shown in Fig. 2, in which Panel A shows that, after MCM8-siRNA knocks down MCM8 in THP-1 cells, the release of IFN-β increases significantly (P < 0.001) compared with a control group (corresponding to Ctrl siRNA in the result graph); and Panel B shows the results of knocking down MCM8 in THP-1 cells and subsequently stimulating the cells with the virus SeV, poly(dA:dT) and the bacterial toxin LPS, respectively. Compared with the control group (corresponding to NC SiRNA in the result graph), the expression of IFN-β increases significantly with P < 0.01. The above results show that, compared with the control group, the MCM8-knockdown group produces more IFN-I in response to the stimulations, which is statistically significant (P < 0.01) to extremely significant (P < 0.001) (in Fig. 2, "**" indicates P < 0.01; and "***" indicates p < 0.001, the same below), indicating that MCM8 knockdown can directly lead to the spontaneous release of IFN-I.

### Embodiment 4: The signaling pathway related to the change of IFN-I expression caused by MCM8 lies in cGAS-STING.

In order to explore the pathway by which the spontaneously released/increased IFN-I is produced, the inventors separately knocked out various receptors that might affect the release of IFN-I, including cGAS, STING, RIG-I and IFI16, from THP-1 cells by means of sgRNA. Similarly, the supernatant described in Example 3 was collected and the release of IFN-β was detected with an IFN-β-specific ELISA kit. The results are as shown in Panel C, Fig. 2, wherein the columns indicate, from left to right, ctrl siRNA blank control group, MCM8 siRNA control group, ctrl siRNA groups with corresponding receptors knocked out, and MCM8 siRNA groups with corresponding receptors knocked out. The data of ctrl siRNA blank control group and MCM8 siRNA control group were different due to the different cell culture time and batch during the experiment. When cGAS or STING was knocked out, the spontaneous release of IFN-I was inhibited, while the other receptors RIG-I and IFI16 were not inhibited (on the contrary, the effect was opposite to that of MCM8-knockdown), indicating that MCM8, cGAS and STING are the targets in the same pathway related to the production of IFN-I, while MCM8 affects the upstream of this pathway and cGAS and STING affects the downstream of this pathway. Similarly, by further stimulating the above cells with SeV, poly(dA:dT) and LPS, it is found that where cGAS or STING is knocked out, the increase of IFN-I caused by MCM8-knockdown is also blocked in response to stimulation.

### Embodiment 5: Decreased MCM8 expression leads to a significant increase of mitochondrial DNA in cytoplasm.

Since cGAS-STING is an important DNA recognition receptor in cytoplasm, the findings in Embodiment 4 also suggests that MCM8-knockdown may be related to the production of cytoplasmic DNA. Therefore, in order to explore whether DNA is produced in cytoplasm and the source, the inventors further knocked down MCM8 in THP-1 cells by transfection with MCM8-specific siRNA and stimulated the cells with SeV or poly(dA:dT). The stimulated cells were subjected to nuclear-cytoplasmic separation (Beyotime nuclear protein and cytoplasmic protein extraction kit was used for the nuclear-cytoplasmic separation herein, and the operation followed the instructions, the same below), and the cytoplasmic DNA was extracted for detecting the source of cytoplasmic DNA by qPCR. It was found from the results that the cytoplasmic DNA was mainly composed of mitochondrial DNA and a small amount of nuclear DNA, and the mitochondrial DNA in the cytoplasm significantly increased upon MCM8-knockdown (see Panel A, Fig. 3, wherein D-loop represents mitochondrial DNA).

In order to further verify this finding, the inventors divided the PBMCs (peripheral blood mononuclear cells) from patients into two groups by sequencing, namely, those carrying normal MCM8 gene (A276, represented by MCM8 ref in the results) and those carrying mutant MCM8 gene (P276, represented by MCM8 mut in the results, and as mentioned above, this mutation reduced the expression of MCM8). The two groups of PBMCs were both stimulated by LPS, SeV or poly(dA:dT), and the cytoplasmic DNAs were then extracted separately, and the DNA sources thereof were detected by Q-PCR. It was found from the results that, in the cytoplasm of Kawasaki disease patients in the mutant group, the amount of mitochondrial DNA increased significantly (see Panel B, Fig. 3). The results demonstrated once again that the reduced MCM8 expression level caused by either MCM8-knockdown or MCM8 mutation would result in accumulation of mitochondrial DNA (mtDNA) in cytoplasm, thereby activating cGAS-STING-mediated IFN-I pathway.

### Embodiment 6: Functional defect in MCM8 activates cGAS-STING-IFN-I signaling pathway by increasing the amount of cytoplasmic mtDNA.

To further verify the mechanism of MCM8-cGAS-STING pathway, the inventors knocked down MCM8 in THP-1 cells, stimulated the cells with SeV, and then treated the cells with MTP complex pore inhibitor cyclosporine A (CsA) (2.5-5 mM) to inhibit the release of mitochondrial DNA. It was found from the results that IFN-β increased significantly when SeV stimulation was applied and no CsA was given in the case of MCM8-knockdown, whereas IFN-β did not increase when SeV stimulation was applied likewise and CsA was given in the same case of MCM8-knockdown (see Panel C, Fig. 3), indicating that the regulatory function of MCM8 in the latter case on IFN-I was significantly inhibited, that is, the function of MCM8 in regulating IFN-I was affected by the amount of mitochondrial DNA, and the production of IFN-I was inhibited when mitochondrial DNA in cytoplasm did not increase in quantity or aggregate. The results were similar when the mitochondria in the cells were removed by EtBr treatment. The process was as follows: THP-1 cells were treated with EtBr to completely remove the mitochondrial DNAs in the cells to construct mitochondrion-free ρ0 cells, MCM8 was knocked down in normal THP-1 or ρ0 THP-1, the cells were then stimulated with SeV, the expressions of IFN-α and IFN-β were detected by Q-PCR, and the level of pIRF3 (IRF3 protein activated by phosphorylation) was detected by western blot. The results showed that in the mitochondrion-free cells (deplete-mito), the function of MCM8 in regulating IFN-I was also blocked (see Panels D and E, Fig. 3). In Panel D, four columns are taken as a group and represent, from left to right, ctrl siRNA blank control group, MCM8 siRNA control group, ctrl siRNA group of mitochondrion-free cells, and MCM8 siRNA group of mitochondrion-free cells. Panel E shows that the expression of pIRF3 protein, a downstream product of cGAS-STING signaling pathway, is also significantly inhibited, indicating that the production or aggregation of cytoplasmic mitochondrial DNA is a target upstream of the cGAS-STING signaling pathway. In conclusion, MCM8-knockdown acts to increase the expression of IFN-I by promoting the aggregation of cytoplasmic mtDNA. On the contrary, drugs that inhibits or eliminats mtDNA can block the function of MCM8 in regulating IFN-I, and MCM8-knockdown activates cGAS-STING-IFN-I signaling pathway by increasing the amount of cytoplasmic mtDNA.

### Embodiment 7: MCM8 participates in mitophagy by regulating the activation of LC3.

Based on the findings in Embodiment 6, MCM8 is related to the amount of cytoplasmic mtDNA and cGAS-STING-IFN-I signaling pathway, while the amount of mtDNA is related to mitophagy, and therefore, the experiment was further carried out as follows. Experiment of MCM8-knockdown inhibiting mitophagy-related proteins: in MCM8-knockdown THP-1 cells, detection indicators related to mitophagy were studied. The cells were stimulated or not stimulated with LPS and SeV, respectively, and the expressions of mitochondrial LC3 and other mitochondrial proteins TOM20, MFN2, PINK1 and COXIV were detected by western blot. It was found from the results that, compared with the control group (Ctrl siRNA), MCM8-knockdown could inhibit the activation of mitochondrial LC3 and increase the amounts of mitochondrial proteins (TOM20, MFN2, PINK1, and COXIV) (see Panel A, Fig. 4). Correspondingly, MCM8 (Flag-MCM8) was overexpressed in Hela cells, the cells were stimulated or not stimulated with LPS and SeV, respectively, and the expressions of mitochondrial LC3 and other mitochondrial proteins (TOM20, MFN2, PINK1, and COXIV) were detected by western blot. It was found from the results that, compared with the control group (EV), MCM8-overexpression promoted the activation of LC3 more obviously, and thus inhibited the increase of mitochondrial proteins (TOM20, MFN2, PINK1, and COXIV) (see Panel B, Fig. 4).

In order to further study the effect of MCM8 on mitophagy, the inventors knocked down MCM8 with siRNA in Hela cells, co-stained LC3 and mitotracker (a mitochondrial marker) by immunofluorescence staining, and finally observed the co-localization of LC3 and mitotracker by a confocal immunofluorescence microscope. The specific process for immunofluorescence involved fluorescent secondary antibodies for immunofluorescence, i.e., gout anti-Mouse 488, gout anti-Mouse 594, gout anti-Mouse 633, gout anti-Rabbit 488, Gout anti-Rabbit 594, Gout anti-Rat 488, and Gout anti-Rat 594, and DAPI, all of which were purchased from Life Technology. Buffer preparation: blocking buffer was PBS containing 0.3% Triton X100 and 5% goat serum, and antibody dilution was PBS containing 0.3% Triton X100 and 1% BSA. After the cells to be detected were prepared ready, a cell supernatant was removed, and the cells were washed with PBS; the supernatant was removed and the cells were fixed with 4% PFA for 15 minutes; the supernatant was removed and the cells were washed 3 times with PBS for 5 minutes each time; the supernatant was removed and the cells were blocked with the blocking buffer for 1 h; the supernatant was removed, the antibody for protein detection was diluted 1 : 500 with the corresponding buffer, and incubated with the cells at 4 degrees overnight; the supernatant was removed and the cells were washed 3 times with PBS for 5 minutes each time; the cells were added with diluted fluorescent secondary antibody (diluted at 1 : 1000) incubated at room temperature for 2 hours; the cells were washed three times with PBS for 5 minutes each time; and the supernatant was removed and a mounting medium containing an antifade agent and DAPI was used for mounting, for later use. The results were shown in Panel C, Fig. 4, which shows that in the control group, there was a significant co-localization signal between LC3 and mitochondria, whereas the co-localization signal between LC3 and mitochondria was significantly reduced when MCM8 was knocked down (MCM8 siRNA in the panel), thus indicating that MCM8-knockdown could significantly inhibit the co-localization of LC3 and mitotracker.

In addition, after MCM8 in THP-1 cells was knocked down, the number of mitophagy was detected by immunoelectron microscopy. It was found from the results that the structures formed by mitophagosomes were significantly reduced in the MCM8-knockdown cells (see Panel D, Fig. 4). Furthermore, the results of further quantitative statistical analysis based on the results of immunoelectron microscopy in Panel D also showed that after MCM8 knockdown, the number of mitophagy in THP-1 cells decreased significantly (see Panel E, Fig. 4), indicating that immunoelectron microscopy more intuitively showed that after MCM8 knockdown, the number of mitophagosomes formed in THP-1 cells decreased significantly (see Panels D and E, Fig. 4).

Furthermore, the MCM8-knockdown THP-1 cells were stimulated or not stimulated with SeV and LPS, respectively, and treated with or without the mitophagosome formation inhibitor Mdivi, and the cytoplasmic DNA was extracted, and the content of mitochondrial DNA in cytoplasm and the expressions of IFNα and IFNβ were detected by Q-PCR. The results showed that the mtDNA aggregation exhibited in MCM8-knockdown cells was similar to that in the control group treated with the mitophagosome formation inhibitor Mdivi (see Panel F, Fig. 4), and the MCM8-knockdown cells showed an increase in the expression levels of IFNα and IFNβ, similarly to that of the control group treated with the mitophagosome formation inhibitor Mdivi (see Panel G, Fig. 4). It was suggested that MCM8-knockdown inhibited the formation of mitophagosomes, thus affecting the function in regulating IFN-I.

The above results suggest that the under-expression of MCM8 leads to the inhibition of the formation of mitophagosomes, which in turn leads to the inhibition of the timely elimination of damaged mitochondria, thus causing more damaged mitochondrial DNAs to accumulate in cytoplasm. In conjunction with the foregoing Embodiments, it could be proved that the under-expression of MCM8 leads to reduced binding with LC3 mitochondria, and the reduced formation of mitophagosomes leads to mitochondrial DNA aggregation, which activates cGAS-STING pathway and promotes the expression of IFN-I, thus promoting the occurrence and development of various diseases such as Kawasaki disease.

### Embodiment 8: MCM8 with LIR motif can directly bind to LC3.

Proteins that directly bind to LC3 generally have a LIR motif, the inventors, when predicting with website whether MCM8 has a LIR motif, find that the sequence between the sites MCM8 272-277 is LIR motif. According to the functional domain of MCM8, the inventors constructed truncated mutants in various truncated forms of MCM8 and detected the results of the binding thereof to LC3. It was found from the results that all the MCM8 containing LIR motif sequence (including full-length MCM8 and truncated MCM8) could bind to LC3, while the MCM8 without LIR motif lost the function of binding to LC3 (see Panel A, Fig. 5, in which FL represents full-length MCM8 protein; dMCM represents MCM8 protein with MCM removed but LIR motif sequence retained; dLIR represents MCM8 protein with LIR motif sequence removed; dN represents MCM8 protein with the N-terminal sequence of MCM removed but the LIR motif sequence retained; and LIR motif sequence refers to RSFTAL). Further mass spectrometry experiments also confirmed that MCM8 could directly bind to LC3.

In addition, different members of the MCM family were further compared, and it was found that only MCM8 and MCM9 have similar LIR motifs, and only MCM8 has a more classic LIR motif (see Panel B, Fig. 5). By means of 3D structure prediction, the binding of MCM8 LIR motif to LC3 was simulated (see Panels C and D, Fig. 5). Interestingly, the point mutation P276 in MCM8 found by the inventors in patients with Kawasaki disease was located on LIR motif. After mutation (A276P), the binding ability of MCM8 to LC3 was greatly reduced (see Panel E, Fig. 5, the α-LC3 band in the IP Flag group disappears).

Furthermore, human PBMCs with normal A276 site and mutated P276 site were extracted, and the cells were stimulated or not stimulated with LPS, SeV and poly(dA:dT), respectively, and the cytoplasmic DNA was extracted, and the content of mitochondrial DNA in cytoplasm was detected by Q-PCR. It was found from the results that when the PBMCs from the patients with Kawasaki disease were stimulated with LPS, SeV or poly(dA:dT), the point mutation P276 in MCM8 resulted in more aggregation of mitochondrial DNA in cytoplasm (see Panel F, Fig. 5).

In conclusion, all the results shows that LIR motif (especially the fragment whose amino acid sequence is RSFTAL at positions 272-277) is essential for the formation of mitophagosomes and the reduction of mitochondrial DNA aggregation in cytoplasm. Therefore, the technical means which utilizes LIR motif or the point mutant thereof as a target provides an optional basis for the diagnosis, prevention and/or treatment of mitophagy-related diseases (e.g., cardiovascular diseases and neuropathy) and diseases caused by abnormal activation of cGAS-STING-IFN-I, the downstream pathway of mitophagy formation dysfunction as demonstrated above (including inflammatory diseases, autoimmune diseases, cancers, and aging-related diseases).

### Embodiment 9: Level of IFN-I, cardiovascular injury and verification of anti-IFNAR antibody effect in MCM8-knockout animal model

To further confirm the effects of MCM8 and IFN-I on the occurrence and development of Kawasaki disease on the physiological level. The inventors constructed MCM8-knockout mice by means of CRISPR/cas9 technology and established a Kawasaki mice model by intraperitoneal injection of LCWE (*Lactobacillus casei* cell wall extract) with help from Nanjing Model Animal. At Day 5 (acute phase) and Day 14 (subacute phase) of modeling, the inflammatory factors in the serum of the mice were detected using a mice 13-factor inflammation CBA kit from Biolegend according to the operation instructions. It was found from the results that in the acute phase, compared with the control group, the expression levels of IFN-β (representing IFN-I), IFN-γ, inflammatory factor TNFα and MCP-1 in the MCM8-knockout group all significantly increased; and in the subacute phase, the expression levels of IFN-β (representing IFN-I), IFN-γ, inflammatory factor TNFα, and MCP-1 all also increased in the MCM8-knockout group, and only the increase of IFN-β and MCP-1 was significantly different from that in the control group (see Panel A, Fig. 6). Therefore, it was indicated that the expression level of IFN-I in MCM8-knockout mice was significantly increased in both the acute and subacute phases of the Kawasaki disease model, demonstrating once again that the defects such as reduced or deficient expression of MCM8 were the key factors leading to the production of IFN-I and the occurrence or development of the disease.

In addition, at Day 14 of modeling, the mice were subjected to cardiac perfusion with PBS and 4% PFA for washing away blood cells, the hearts of the mice were embedded in wax and sectioned, and performed staining with hematoxylin and eosin (H&E) (or HE staining for short). The specific method for HE staining involved: fixing an animal tissue in 4% paraformaldehyde (PFA) for 24 h, sequentially soaking the animal tissue in 50%, 60%, 70%, 80%, 90% and 100% ethanol for dehydration, hyalinizing the animal tissue with xylene twice, embedding in paraffin, and cutting into 4 µm sections; after baking at 60°C, subjecting the paraffin sections sequentially to xylene twice for 15 min each time to dewax, 100% and 70% ethanol twice to rehydrate, and soaking in distilled water twice; after staining with hematoxylin at room temperature for 6 min, washing with running water to remove the floating color; subjecting to color separation with 1% hydrochloric acid alcohol for 3 s, placing in tap water for bluing by running water for 5 min; staining with eosin for 3 min, subjecting to 70% and 100% ethanol twice sequentially for dehydration, hyalinizing with xylene twice, mounting with neutral resin and observing under microscope. The HE staining results showed that there was more severe immune cell infiltration in the heart of the MCM8-knockout mice (see Panel B, Fig. 6), suggesting more severe vascular inflammation and vascular injury. In addition, a Kawasaki disease mice model induced with LCWE was re-taken and treated with anti-IFNAR antibody (Leinco Technologies, article No. I-401, an IFN-α receptor antibody), the hearts of mice at 14 days of modeling were taken and re-subjected to the steps of "embedding paraffin sections after washing with PBS and 4% PFA and observing vascular inflammation in the mice by HE staining". The HE staining results showed significantly mitigated vasculitis in the WT and MCM8-knockout mice after IFNAR antibody treatment, and the infiltration of immune cells was mitigated to close to that of the unmodeled group (see Panel C, Fig. 6). In addition, the results of clinical score statistics and quantitative detection on Panels B and C of Fig. 6 also showed that the cardiac vasculitis in the MCM8-knockout mice treated with anti-IFNAR was significantly improved (see Panel D, Fig. 6). Therefore, it is demonstrated that the regulation of cGAS-STING-IFN-I pathway by affecting the targets in the MCM8-related pathway, e.g., by giving anti-IFNAR antibody, is a feasible prevention and treatment solution to diseases such as vasculitis.

Furthermore, the expression of IFN-α in heart tissue was detected by IHC staining. The specific method for IHC staining involved: baking sections at 58°C for one hour, subjecting to xylene I, xylene II, 100%, 90%, 80% and 70% ethanol, distilled water and distilled water sequentially to dewax; then putting the sections into an antigen retrieval solution, boiling in a microwave oven at a high power for 3 min and then at a medium or low power for 15 minutes for antigen retrieval; soaking slides in 3% hydrogen peroxide for 20 minutes; washing three times with PBS; adding non-immune goat serum and incubating at room temperature for half an hour; putting the slides into a wet box, adding a primary antibody, and leaving to stand at 4 degrees overnight; and the next day, taking out the slides, rewarming, and then washing three times with PBS; adding a secondary antibody and incubating at room temperature for 1 hour, and washing three times with PBS; adding DAB developer, and observing color development under microscope and terminating duly; adding hematoxylin for staining for 2 minutes, rinsing with distilled water, bluing with a bluing solution for 5 minutes, and rinsing with tap water for 5-10 minutes; and soaking sequentially in 70%, 80%, 90% and 100% ethanol for dehydration, and hyalinizing with xylene twice, mounting with neutral resin. The results from the immunohistochemical staining images (see Panel E, Fig. 6) and the quantitative analysis graph (see Panel F, Fig. 6) showed that after MCM8 knockout, IFN-α in the mice was significantly up-regulated, which was consistent with the results of the other previous examples.

### Embodiment 10: MCM8 Deletion leads to pulmonary vasculitis.

MCM8-knockout mice (including MCM8+/- heterozygous mice and MCM8-/- homozygous mice) constructed by means of CRISPR/cas9 technology were fed together with WT wild-type mice with normal diet for 32 weeks in SPF (aseptic) feeding environment. 4 mice randomly from the WT and MCM8-knockout mice, respectively, were sacrificed, and taken their lungs for paraffin embedding and HE staining. Results: Panel A of Fig. 7 shows the HE pathological panorama and enlargement of pathological sites of the lungs in MCM8-/- mice, and it could be clearly observed that there are dark colored immune cells infiltrating around blood vessels. Panel B of Fig. 7 shows the HE pathological panorama and local enlargement of the lungs in WT mice, and it could be observed that WT mice have the clear and complete structures which are relatively healthy without inflammatory infiltration. Therefore, it is indicated that MCM8-knockout mice, especially MCM8-/- mice, would have spontaneous pulmonary vasculitis when they are old, which not only indicates that MCM8 is a target for pulmonary vasculitis, but also suggests that a model of pulmonary vasculitis can be prepared in this way.

In conclusion, Embodiments 1-9 above take Kawasaki disease as an example to illustrate that the expression level of MCM8 is reduced due to the defect in MCM8 gene, which affects the binding of MCM8 to mitochondrial LC3 and further affects the formation of mitophagosomes, leading to mitochondrial DNA aggregation in cytoplasm, thereby activating cGAS-STING-IFN-I signaling pathway, making the expression of IFN-I abnormally increase, promoting the occurrence and development of vasculitis diseases including Kawasaki disease, cardiac vasculitis, and hematological vasculitis. Embodiment 10 further demonstrates that the defect in MCM8 gene leads to the occurrence and development of pulmonary vasculitis, and thus it can be considered that the defect in MCM8 is related to vasculitis in multiple parts of the whole body, and MCM8 can be used as a target for the diagnosis and treatment of vasculitis.

Embodiments 11-14 below further illustrates by taking more disease models that the decrease of MCM8 gene expression or the deletion of MCM8 gene would also lead to other diseases associated with dysfunctional mitophagy and diseases associated with abnormal activation of cGAS-STING-IFN-I signaling pathway.

### Embodiment 11: MCM8 deletion aggravates lung inflammation.

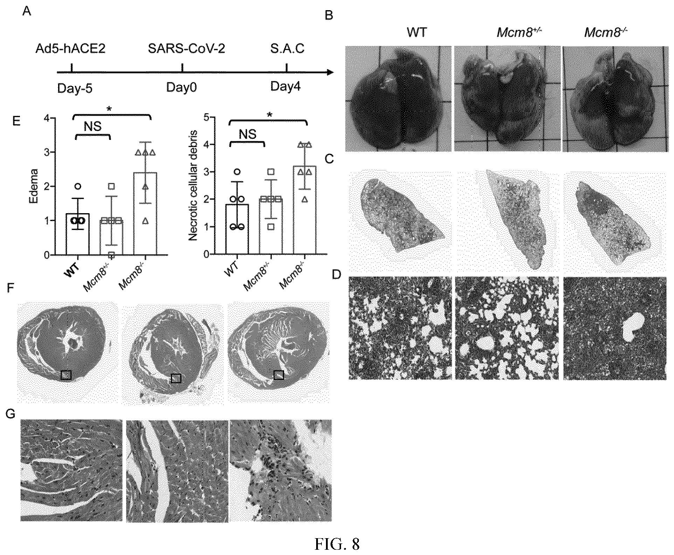

MCM8-knockout mice (including MCM8+/- heterozygous mice and MCM8-/- homozygous mice) were constructed by means of CRISPR/cas9 technology. Next, 8-week-old mice were used to construct a COVID-19s infection model. The process included the steps of: first, infecting the mice with adenovirus that overexpresses human ACE2 by nasal drip; 5 days after nasal drip, infecting the mice with COVID-19 (Corona Virus Disease 2019, provided by Guangzhou Medical University); 4 days after infecting, the mice were sacrificed and taken the lungs for photography, fixation and HE staining (see Panel A of Fig. 8, a graphical representation of the method for modeling the COVID-19 infection mice model).

The results shows: from the images of the lungs of the mice in Panel B of Fig. 8, it can be seen that the lungs of both the COVID-19 model WT and MCM8knockout mice have severe injuries, indicating successful modeling. It is not enough to judge the severity of injury from the lung images alone, so the lungs and hearts of the mice were further embedded and stained with H&E.

From the panorama of HE pathological sections of the lungs of the mice in Panel C of Fig. 8, it can be seen that compared with the HE pathological sections of the lungs of WT and MCM8+/- mice, those of the MCM8-/- mice shows severer lung injury and structural damage. Panel D of Fig. 8 show the enlargement of the injured site, and it is observed that compared with WT and MCM8+/- mice, the MCM8-/- mice have severer edema and more necrotic cellular debris. Panel E of Fig. 8 shows the score statistics of edema and cellular necrosis in the WT, MCM8+/- mice and MCM8-/- mice based on HE pathological sections. From Panels F and G of Fig. 8, the panoramic and enlarged views of the HE pathological sections of the hearts of the mice, it can be seen that compared with the HE pathological sections of the hearts of WT and MCM8+/- mice, those of MCM8-/- mice show obvious inflammatory infiltration, indicating that MCM8-/- mice have severe pneumonia, which has obviously affected the heart.

Therefore, it is indicated that the defect in MCM8 gene aggravates pneumonia, especially severe pneumonia, and thus MCM8 can also be used as a target for the diagnosis and treatment of pneumonia or severe pneumonia and a target for the animal model construction.

### Embodiment 12: MCM8 deletion significantly increases the severity of renal injury and SLE.

MCM8-knockout mice were constructed by means of CRISPR/cas9 technology, and were further established as a lupus nephritis mice model by intraperitoneal injection of 0.5 mg pristane. The survival time, autoantibody concentration and HE pathological sections of the mice were detected. It was found from the results that compared with wild-type mice, MCM8-deficient mice were obviously more likely to die of severe kidney injury during modeling (see Panel a, Fig. 9). In addition, the concentration of anti-dsDNA antibody in the serum of mice was detected 2 months after intraperitoneal injection of pristane, and it was also found that the anti-self-dsDNA antibody in the serum of the MCM8-deficient mice increased (see Panel b, Fig. 9). In addition, 4 months after injection of pristane, HE pathological sections showed that in addition to severe injury observed in kidney, injury was also likewise observed in the lungs, indicating that lungs had been seriously affected in the SLE disease model. From Panel c of Fig. 9, the HE pathological sections of the lung and the enlargement of the lesion sites in mice, it could be seen that compared with WT mice, MCM8-knockout mice had severer symptoms similar to interstitial pneumonia and the lung structure was seriously damaged. As could be seen from Panel d of Fig. 9, the MCM8-knockout mice showed coagulative necrosis or tissue autolysis phenomena, some renal tubular nuclei were reduced or disappeared, and local lesions showed hemorrhage and protein casts. The above results show that in addition to the pneumonia discussed in Embodiment 11, MCM8-knockout also leads to more obvious and severer manifestations of nephritis and lupus erythematosus (SLE), and thus MCM8 is also useful as a target for the diagnosis and treatment of nephritis and systemic lupus erythematosus disease and as a target for the animal model construction. As mentioned in the Background, the pathological basis of systemic lupus erythematosus belongs to systemic vasculitis. Therefore, it is demonstrated once again that the defect in MCM8 gene or protein is a sign of many vasculitis diseases.

### Embodiment 13: MCM8 deletion promotes steatohepatitis.

MCM8-knockout mice were constructed by means of CRISPR/cas9 technology. The livers of 32-week-old WT and MCM8-/- knockout mice were fixed, embedded with paraffin and sectioned, and the sections were subjected to HE staining for detection of the pathological condition. It was found from the results that compared with the control group, the MCM8-/- liver had obvious vacuolar adipose and inflammatory cell infiltration (see Fig. 10). Therefore, it is indicated that aged MCM8-knockout mice could spontaneously produce fatty liver phenotype, and MCM8 is useful as a target for the diagnosis and treatment of fatty liver and as a target for the animal model construction.

### Embodiment 14: MCM8-knockout causes corneal whitening in mice.

MCM8-knockout mice were constructed by means of CRISPR/cas9 technology, and the eyes of 32-week-old mice were observed. It was found from the results that MCM8-/- knockout mice had corneal whitening in the eyes (see Panels A, B and C, Fig. 11), and small eyes and eye absence in some cases, wherein the case of eye absence was shown in Panel D of Fig. 11. Therefore, MCM8 is useful as a target for the diagnosis and treatment of cataract and as a target for the animal model construction.

### Embodiment 15: NO-induced binding of TRIM21 with MCM8 promotes the ubiquitination and nuclear export of MCM8for functioning.

MCM8, as a chromatin maintenance gene, is mainly located in the nucleus, while the above studies all focus on the mechanism related to extracellular signaling pathway. On this basis, in order to further extensively study the mechanism of how MCM8 functions, including whether it is necessary to export from the nucleus to function, how the aforementioned signaling pathway and the targets thereof (including binding to mitochondrial LC3, initiation of mitophagosome formation, elimination of cytoplasmic damaged mitochondrial DNA in cytoplasm, and cGas-STING-type I signaling pathway) to be regulated, among others, the following experiments were carried out.

The cells were treated with leptomycin B, a nuclear export inhibitor, to inhibit the protein shuttle out of the nucleus, and the expressions of MCM8 in the cytoplasm (cyto) and the nucleus (nuc) were respectively detected by nuclear-cytoplasmic separation. The results showed that compared with the control group (Vehicle), upon stimulation with SeV and LPS, some of MCM8 was exported from the nucleus (increased expression of MCM8 in the cytoplasm was detected), whereas after the addition of leptomycin B, the nuclear export of MCM8 was significantly inhibited (no increase in the expression of MCM8 in the cytoplasm), see Panel a, Fig. 12. In addition, the ubiquitination modification (HA-Ubiqutin) of MCM8 in the cytoplasm and the nucleus was detected by co-immunoprecipitation, and it was found from the results that compared with the signals in the nucleus, upon stimulation with LPS and poly(dA:dT), there were significantly more ubiquitination modifications in the cytoplasm (see Panel b, Fig. 12). However, when TAK-243, a ubiquitin ligase inhibitor, was added to inhibit ubiquitination, the nuclear export of MCM8 was obviously inhibited, which was reflected in that: MCM8 in the cytoplasm increased upon stimulation with LPS and poly(dA:dT) without TAK treatment detected after nuclear-cytoplasmic separation; whereas after TAK treatment, MCM8 in the cytoplasm did not increase upon stimulation, see Panel c, Fig. 12. It could be seen that ubiquitination was essential for the nuclear export of MCM8.

Further, HeLa cells were transfected with Flag-TRIM21 and HA-MCM8 and subjected to co-immunoprecipitation analysis. The results showed that MCM8 could bind to E3 ubiquitin ligase TRIM21, and the binding increased upon stimulation with LPS or poly(dA:dT) (see Panel d, Fig. 12).

In addition, after the cells were treated with NO scavenger 1400W, it was found that the ubiquitination of MCM8 mediated by TRIM21 was significantly inhibited (see Panel e, Fig. 12). Furthermore, upon stimulation with LPS and poly(dA:dT), the nuclear export of MCM8 was also inhibited by 1400W (see Panel f, Fig. 12).

To sum up, the cells under stress produce NO, which promote the translocation of TRIM21 into the nucleus, after which TRIM21 mediates the ubiquitination and the nuclear export of MCM8. After being exported from the nucleus, MCM8 bind to the damaged mitochondria, LC3s are recruited, and the damaged mitochondria are eliminated by mitophagy. Once MCM8 is deficient or mutated, mitophagy is impaired, and the damaged mitochondria would not be eliminated. Mitochondrial DNA is released into the cytoplasm via BAX/BAK or VDAC1 pores, and mtDNA in the cytoplasm is recognized by cGAS, activating IFN-I signaling pathway via cGAS-STING signaling pathway to release IFN-I, resulting in vascular injury and vasculitis (see Panel g, Fig. 12).

Therefore, the results of Embodiments 1-15 comprehensively suggest that when oxidative stress caused by mitochondrial damage produce NO signal, the NO signal can promote TRIM21-mediated ubiquitination of MCM8, so that MCM8 would be exported from the nucleus to exert the function in the other signaling pathways that have been verified hereinbefore and to inhibit the occurrence of many diseases described above, e.g. vasculitis diseases such as Kawasaki disease, systemic lupus erythematosus, cardiovascular inflammation, pulmonary vasculitis or vasculitis with abnormal blood composition, pneumonia or severe pneumonia, nephritis, fatty liver, and eye injury.

Finally, since the expression of MCM8 is reduced (knocked down or silenced), the formation of mitophagosomes is hindered, mitochondrial DNA in cytoplasm increases, cGAS-STING signaling pathway is activated, and IFN-I signaling pathway is abnormally activated. Therefore, mRNA, protein or functional fragment products of MCM8, including full-length or truncated, may be prepared to interact with the targets in the signaling pathway discovered according to the present disclosure. Therefore, exogenous mRNA, protein or functional fragment products of MCM8 are effective means capable of preventing and treating the diseases mentioned in the present disclosure. Therefore, Embodiments 16 and 17 are provided to illustratively demonstrate the effective products for prevention of disease targeting MCM8.

### Embodiment 16: Preparation of mRNA product of MCM8

1. Full-length mRNA of MCM8 (SEQ ID NO: 1), or LIR motif functional fragment of MCM8 (SEQ ID NO: 3), or other truncated MCM8 gene sequence containing LIR motif functional fragment (e.g., SEQ ID NO: 5 or SEQ ID NO: 6, or any other 5' or 3' terminal truncated sequence of MCM8 gene containing LIR motif functional fragment) is synthesized,
   wherein the mRNA is optionally synthesized by *in vitro* transcription using a conventional kit commercially available in the art or expressed using plasmids, lentiviruses, adenoviruses, or *Escherichia coli* vectors commonly used in the art; in addition, the mRNA can be further purified to obtain a purified mRNA, and the method for purification comprises one of or any combination of lithium chloride/ethanol precipitation, centrifugal column, chlorine extraction/ethanol precipitation, gel purification, or high performance liquid chromatography purification; and
2. The mRNA of interest and additives (optionally including a stabilizer, a pH regulator, a buffer, a lipid polymer, a solvent, etc., wherein the solvent is selected from the group consisting of DMSO, ethanol, polyethylene glycol, water, etc.) are combined by a conventional method in the art to prepare a product for exogenously delivering the mRNA sequence, wherein the product may be selected from, for example, liposomes or nano-liposomes.

### Embodiment 17: Preparation of polypeptide or protein product of MCM8

1. Full-length protein of MCM8 (SEQ ID NO: 2), or LIR motif functional fragment (SEQ ID NO: 4), or other truncated sequence of MCM8 protein containing LIR motif functional fragment (e.g., SEQ ID NO: 7 or SEQ ID NO: 8, or any other N-terminal or C-terminal truncated sequence of MCM8 protein containing LIR motif functional fragment) is synthesized or purified by a conventional method; and
2. A product for exogenously delivering the above polypeptide or protein is prepared by a conventional method in the art, e.g., by adding pharmaceutically acceptable adjuvants to prepare fine powders or freeze-dried powders, microemulsions, eye drops or eye ointments, solutions (such as oral solutions and injections), liposomes, transdermal absorption preparations (ointments or patches or gels), nasal drops, pulmonary inhalation preparations (such as inhalation powders), sustained and controlled release preparations (microsphere injections and implants), etc.

The various technical features of the above embodiments can be combined arbitrarily, unless otherwise specified. For the sake of concise description, it is not intended to describe exhaustively all possible combinations of the various technical features of the above embodiments. However, the combinations of these technical features are considered as falling within the scope of the description.

The above embodiments with specific and detailed description merely represent several embodiments according to the present disclosure, but they are not intended to be construed as a limitation on the scope of the present disclosure. It should be understood that for those of ordinary skill in the art, variations and improvements can be made without departing from the concept of the present disclosure, all of which fall within the scope of the present disclosure. Therefore, the scope of the present disclosure should be based on the appended claims.

## Claims

1. Use of a reagent for quantitatively detecting a gene expression or protein expression or protein activity of MCM8 in the preparation of a product for the diagnosis of a disease, wherein the disease is a disease caused by dysfunctional mitophagy and/or a disease caused by abnormal activation of cGAS-STING-IFN-I signaling pathway;
wherein if the gene expression or protein expression or protein activity of MCM8 is lower than a reference level, the disease is diagnosed; and the reference level represents the gene expression level or protein expression level or protein activity level of MCM8 in a subject in the same range of age who does not have the disease;
the product comprises a kit, a diagnostic reagent, a detection reagent, a gene chip or a protein chip;
the protein activity is preferably a binding activity of MCM8 protein to TRIM21 protein or a binding activity of MCM8 protein to mitochondrial LC3 protein;
more preferably, when detecting the binding activity of MCM8 protein to TRIM21 protein, the detection method for the binding activity is to detect the ubiquitination level of MCM8 protein; when detecting the binding activity of MCM8 protein to mitochondrial LC3 protein, the detection method for the binding activity is to detect the binding activity of LIR motif sequence shown in amino acid sequence SEQ ID NO: 4 in MCM8 protein to mitochondrial LC3 protein;
preferably, the product is configured for performing one or more of the detection methods selected from the group consisting of: polymerase chain reaction, micro digital polymerase chain reaction, fluorescence polymerase chain reaction, loop-mediated isothermal amplification reaction, enzyme-linked immunosorbent assay, nucleotide sequencing or amino acid sequencing, denaturing gradient gel electrophoresis, nucleic acid typing chip detection, high performance liquid chromatography, in-situ hybridization, biological mass spectrometry, high-resolution melting curve analysis, single-strand conformational isomerism polymorphism analysis, or probe amplification-refractory mutation system analysis;
preferably, the product contains a primer and/or detection probe for detecting MCM8 gene and/or control gene; and/or further contains a sample treatment reagent, including but not limited to a sample lysis reagent, a sample purification reagent and/or a sample nucleic acid extraction reagent; and/or further contains one or more of DNA extraction reagent, dNTP, DNA polymerase, double-strand specific fluorescent dye, and water; and
preferably, the product is used for a detection specimen or test sample comprising one or more of blood, body fluid, urine, tissue and cell samples from a subject; the subject is an adult or a child, preferably a child.

2. Use of MCM8 gene-knockout mice in the preparation of an animal model of a disease, wherein the disease is a disease caused by dysfunctional mitophagy and/or a disease caused by abnormal activation of cGAS-STING-IFN-I signaling pathway.

3. Use of a substance for improving a gene expression or protein expression or protein activity of MCM8 in the preparation of a drug for the prevention and/or treatment of a disease, wherein the disease is a disease caused by dysfunctional mitophagy and/or a disease caused by abnormal activation of cGAS-STING-IFN-I signaling pathway;
the protein activity is preferably a binding activity of MCM8 protein to TRIM21 protein or a binding activity of MCM8 protein to mitochondrial LC3 protein;
preferably, the substance for improving a gene expression or protein expression or protein activity of MCM8 is selected from the group consisting of a micromolecular drug, a polysaccharide drug, a nucleic acid drug, and a polypeptide or protein drug;
more preferably; the substance is one or more of a nucleic acid drug containing an MCM8 expression promoter or enhancer, a nucleic acid drug having any truncated or full-length sequence of MCM8 mRNA containing at least a nucleotide sequence shown in SEQ ID NO: 3, a polypeptide or protein drug having any truncated or full-length sequence of MCM8 protein containing at least an amino acid sequence shown in SEQ ID NO: 4, a nucleic acid drug encoding a polypeptide or protein drug having any truncated or full-length sequence of MCM8 protein containing at least an amino acid sequence shown in SEQ ID NO: 4, and a molecular glue capable of binding MCM8 protein to mitochondrial LC3 protein;
wherein the nucleic acid drug having any truncated or full-length sequence of MCM8 mRNA containing at least a nucleotide sequence shown in SEQ ID NO: 3 further comprises a DNA sequence in which U in the mRNA is replaced by T or a complementary sequence thereto;
preferably, the drug is prepared in a dosage form suitable for adults or children, preferably a dosage form suitable for children; and/or a dose form suitable for gastrointestinal administration, transdermal administration, ocular administration, nasal administration, or pulmonary administration.

4. A pharmaceutical composition for the prevention and/or treatment of a disease, wherein the disease is a disease caused by dysfunctional mitophagy and/or a disease caused by abnormal activation of cGAS-STING-IFN-I signaling pathway, and the pharmaceutical composition comprises a combination of a) and c) or a combination of a), b) and c):
a) one of or any combination of a drug for improving the gene expression of MCM8, a drug for improving the protein expression of MCM8, a drug for improving the binding of MCM8 protein to TRIM21 protein, a drug for improving the ubiquitination or phosphorylation of MCM8 in nucleus, and a drug for improving the binding of MCM8 protein to mitochondrial LC3 protein,
wherein a) is selected from the group consisting of a micromolecular drug, a polysaccharide drug, a nucleic acid drug, and a polypeptide or protein drug; more preferably, a) is a nucleic acid drug containing an MCM8 expression promoter or enhancer, a nucleic acid drug having any truncated or full-length sequence of MCM8 mRNA containing at least a nucleotide sequence shown in SEQ ID NO: 3, a polypeptide or protein drug having any truncated or full-length sequence of MCM8 protein containing at least an amino acid sequence shown in SEQ ID NO: 4, and a molecular glue capable of binding MCM8 protein to mitochondrial LC3 protein,
wherein the nucleic acid drug having any truncated or full-length sequence of MCM8 mRNA containing at least a nucleotide sequence shown in SEQ ID NO: 3 further comprises a DNA sequence in which U in the mRNA is replaced by T or a complementary sequence thereto;
b) one of or any combination of a drug for reducing mitochondrial DNA aggregation in cytoplasm, a drug for inhibiting cGAS-STING signaling pathway, a drug for inhibiting IFN-I expression or binding to a receptor thereof, and other active drugs capable of preventing or treating the disease,
wherein b), when selected from a drug for inhibiting IFN-I expression or binding to a receptor thereof, is a neutralizing antibody against IFN-I including IFN-α and/or IFN-β, a blocking antibody against IFN-I receptor, or a drug stimulating a subject to produce an antibody against IFN-I; more preferably, the antibody is one or more of Anifrolumab, Rontalizumab, Sifalimumab, AGS-009, IFNα Kinoid, NI-0101, and 1-401; and
c) a delivery vector for a nucleic acid, polypeptide, or protein; and/or a pharmaceutically acceptable carrier.

5. A diagnostic marker for a disease, wherein:
the disease is a disease caused by dysfunctional mitophagy and/or a disease caused by abnormal activation of cGAS-STING-IFN-I signaling pathway;
the diagnostic marker is a mutant MCM8 gene or MCM8 protein; the mutant MCM8 gene has a decreased expression level relative to a reference level, or the mutant MCM8 protein has a decreased expression level relative to a reference level, or the mutant MCM8 protein has a decreased binding activity to TRIM21 protein relative to a reference level, or the mutant MCM8 protein has a decreased binding activity to mitochondrial LC3 protein relative to a reference level; the mutant MCM8 protein having a decreased binding activity to mitochondrial LC3 protein relative to a reference level preferably has a mutation present in LIR motif sequence in MCM8 protein as shown in SEQ ID NO: 4; and the reference level represents the gene expression level or protein expression level or protein activity level of MCM8 in a subject in the same range of age who does not have the disease.

6. The use according to any one of claims 1-3, or the pharmaceutical composition according to claim 4, or the diagnostic marker according to claim 5, wherein the disease caused by dysfunctional mitophagy and/or the disease caused by abnormal activation of cGAS-STING-IFN-I signaling pathway are selected from the group consisting of autoimmune diseases, inflammatory diseases, neurological conditions, aging-related diseases or cancers caused by dysfunctional mitophagy and/or by abnormal activation of cGAS-STING-IFN-I signaling pathway.

7. According to claim 6, wherein the disease caused by dysfunctional mitophagy and/or the disease caused by abnormal activation of cGAS-STING-IFN-I signaling pathway are selected from the group consisting of vasculitis, pneumonia, nephritis, fatty liver, and cataract.

8. According to claim 7, wherein the vasculitis is primary or secondary vasculitis; or the vasculitis is selected from the group consisting of Kawasaki disease, systemic lupus erythematosus, cardiac vasculitis, pulmonary vasculitis, and vasculitis with abnormal blood composition.

9. A diagnostic marker for vasculitis, wherein
the diagnostic marker is MCM8 gene with 826G>C and/or 1094G>A and/or 839C>A and/or 2291C>G mutations; or MCM8 protein with A276P and/or S365N and/or S280C and/or S764A mutations; or other MCM8 protein single mutations or linked mutations except A276P mutation present at positions 272-277 of the MCM8 protein sequence of SEQ ID NO: 2, or gene mutations or linked mutations at the corresponding sites; and preferably, the vasculitis is Kawasaki disease or systemic lupus erythematosus.

10. Use of the diagnostic marker of claim 9 in the preparation of a product for the diagnosis of vasculitis, wherein
the product comprises a kit, a diagnostic reagent, a detection reagent, a gene chip or a protein chip;
the product optionally contains a primer and/or detection probe for detecting MCM8 gene or control gene; and/or contains a sample treatment reagent, including but not limited to a sample lysis reagent, a sample purification reagent and/or a sample nucleic acid extraction reagent; and/or further contains one or more of DNA extraction reagent, dNTP, DNA polymerase, double-strand specific fluorescent dye, and water;
preferably, the product is used for a detection specimen or test sample comprising one or more of blood, body fluid, urine, tissue, and cell samples from a subject to be tested; the subject is an adult or a child, preferably a child; and
preferably, the vasculitis is Kawasaki disease or systemic lupus erythematosus.
